(19) Europäisches Patentamt
European Patent Office
Office européen des brevets

(11) EP 2 369 493 A1

(12) **EUROPEAN PATENT APPLICATION**
published in accordance with Art. 153(4) EPC

(43) Date of publication:
28.09.2011 Bulletin 2011/39

(21) Application number: 09831427.1

(22) Date of filing: 09.10.2009

(51) Int Cl.:
$G06F\ 17/30^{(2006.01)}$  $G06F\ 17/00^{(2006.01)}$
$G06F\ 17/15^{(2006.01)}$  $G06F\ 19/00^{(2011.01)}$
$C12N\ 15/00^{(2006.01)}$  $C40B\ 40/06^{(2006.01)}$

(86) International application number:
PCT/CN2009/074377

(87) International publication number:
WO 2010/066150 (17.06.2010 Gazette 2010/24)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 08.12.2008 CN 200810239284
11.09.2009 CN 200910092412

(71) Applicant: Tsinghua University
Beijing 100084 (CN)

(72) Inventors:
• LI, Shao
Beijing 100084 (CN)
• ZHANG, Ningbo
Beijing 100084 (CN)

(74) Representative: Richebourg, Michel François
Cabinet Michel Richebourg
Le Clos du Golf
69, rue Saint-Simon
42000 Saint-Etienne (FR)

(54) **GENE NETWORK-BASED METHOD FOR CONFIRMING DRUG ACTION**

(57) The invention provides a network-based method for confirming drug action(drug effect, synergistic reaction). The method be carried out by mapping a first drug genes/gene products subset and a second genes/gene products subset in a gene network. The second genes/gene products subset can be a second drug genes/gene products subset or a biological process genes/gene products subset. The invention also provides a tool for preclinical drug screening.

Fig.3

## Description

### Field of the Invention

The present invention relates to a method of network-based identification for drug action (NIDA) and/or synergy effect of medicine combination.

### Background of the invention

Determination of action of medicine is one of the most important basic tasks in medicine research and production, and it has a plurality of aspects, among which a basic one is determination of the treatment effect of a medicine.

Some traditional medicine (particularly Chinese traditional medicine) has good treatment effects on relevant diseases. For example, a traditional Chinese medicine recipe called "Qingluoyin" (QLY) having kuh-seng, caulis sinomenii, golden cypress, and poison yam as its main ingredients has good effect in treating angiogenesis-related diseases, such as arthritis deformans. But QLY has complicated components, with kuh-seng having 54 known components, caulis sinomenii having 45, golden cypress having 46, and poison yam having 30.

Kuh-seng contains Matrine; Sophoridine; Isomatrine; 7,11-Dehydromatrine; Sophocarpine; Isosophocarpine; Sophoramine; $\Delta^7$-Dehydrosophoramine; Sophoranol; 9a-hydroxysophoramine; 5a,9a-dihydroxymatrine; Oxymatrine; N-oxysophocarpine; Sophoranol-N-oxide; N-methylcytisine; Rhombifoline; Lupanine; Anagyrine; Baptifoline; mamanine; Kuraramine; Isokuraramine; Isoanhydroicaritin; Kushenol C; Kushenol G; Isoxanthohumol; Nor-kurarinone; Kurarinone; Iso kurarinone; Kurarinol; Neokurarinol; Nor-kurarinol; Sophoraflavanone; Kushenol A; Kushenol B; Kushenol E; Kushenol F; Kushenol H; Kushenol I; Kushenol J; Kushenol K; Kushenol L; Kushenol M; Kushenol N; Formononetin; Kushenol O; Kuraridin; Kuraridinol; Kushenol D; Trifolirhizin; Kushenin; Maackiain.

Caulis sinomenii contains 8-Oxocanadine; (-)-8-Oxotetrahydropalmatine; Cheilanthifoline; Acetamide, N-(1,7-dimethoxy-2-phenanthrenyl)- (9CI); Caffeine; 1,7-Dimethylxanthine; Acutupyrrocoline; (+)-menisperine; (+)-laurifoline; Dehydrodiscretine; Epiberberine; Palmatine; Menisdaurilide; Aquilegiolide; 2(4H)-Benzofuranone, 5,6,7,7a-tetrahydro-6-hydroxy-, (6R-trans)-; (+)-Dihydroaquilegiolide; 8,14-dihydrosalutaridine; Stepharanine; Bianfugenine; Sinomendine; Magnoflorine; Salicylal dehyde; palmitic acid; 6H-Dibenzo[de,g]quinoline-6-carboxaldehyde, 4,5-dihydro-1,2-dimethoxy-(9CI); (-)-stepholidine; Liriodenine; Allantoin; aristolochic acid; stepharine; N,O-diace-tylmichelalbine; N-acetylstepharine; Michelalbine; acutumidine; dl-Syringeresinol; Syringaresinol; sinoacutine; isosinomenine; (-)- -Sitosterol; stigmasterol; tuduranine; sinactine; bisinomenine; sinomenine (discovered by Ishiwara); Diversine (discovered by Taguchi).

Golden cypress contains phellodensin G; phellodenol D; phellodenol E; obacunone; obaculactone; berberine; Palmatine; Daucosterol; Cyclohexanecarboxylic acid; 3-acetyl-3,4-dihydro-5,6-dimethoxy-1H-2-benzopyran-1-one; quercetin-3-O- -D-galactoside; Dihydrokaempferol; Phellochinin A; (E,E,Z)-N-(2-Methylpropyl)-2,4,8-tetradecatrienamide; N-methylflindersine; Melianone; Phellochin; (-)-Syringaresinol diglucoside; -D-Glucopyranoside,2,6-dimethoxy-4-[tetrahydro-4-[(4-hydroxy-3,5-dimethoxyphenyl)methyl]-3- (hydroxymethyl)-2-furanyl]phenyl, [2R-(2 ,3 ,4 )]- (9CI); Cyclohexanecarboxylic acid; Cathin-6-one; Friedelin; 5-Cyclodecen-1-ol, 4,10-bis(methylene)-7-(1-methylethyl)- (9CI); Niloticin; dihydroniloticin; niloticin acetate; Hispidol B; Piscidinol A; Cneorin NP36; Sylvapine A, -Pinene; -Geraniolene, -Myrcene; Cyclohexene, Limonene; NSC 319644, cis-Carveol; trans-Carveol; (+)-Carvone; trans-Limonene oxide; cis-Limonene oxide; -Elemene; Menisperine; phenllodendrine; ferulic acid; adenosine; Noroxyhydrastinine; jatrorrhizine; Berbithine; Anhydroberberilic acid; and

Poison yam containsspongiosin B, Diospongin B; spongiosin C, Diospongin C; Piperitol; Sesaminone; hypoglaucin G; Dioscin; gracilin; -D-Glucopyranoside; (1R)-1-ethenylhexyl; 6-O- -L-arabinopyranosyl- (9CI); -D-Glucopyranoside, (3 ,22 ,25R)-26-( -D-glucopyranosyloxy)-22-hydroxyfurost-5-en-3-yl ; 2-O-(6-deoxy- -L-mannopyranosyl)-(9CI); -D-Glucopyranoside, (3 ,22 ,25R)-; 26-( -D-glucopyranosyloxy)-22-; hydroxyfurost-5-en-3-yl O-6-deoxy- -L-; mannopyranosyl-(1 2)-O-[6-deoxy- -L-mannopyranosyl-(1 4)]- (9CI); Hypoglaucin ; Hspongipregnoloside A; Spongipregnoloside B; Spongipregnolosides C; Spongipregnolosides D; -D-Glucopyranoside, (1R)-1-ethenylhexyl 6-O- -L-arabinopyranosyl- (9CI); Orbiculatoside B; Dumoside; Trigofoenoside D 1; Zizyvoside I; Glycoside D; Lilioglycoside R; Protogracillin; Methylprotodioscin; Pregnadienolone, 3-O- -gracillimatriose; Gracilline; Lilioglycoside G; Lilioglycoside D; Prosapogenin A; Collettiside III; Dioscine; Polyphyllin III; Doursterol; Sitogluside; diospongins A; (+)-Lirioresinol B; +)-Syringaresinol.

With conventional trying-testing approach, it is very difficult and time/money-consuming to determine the effective components from so many known components. In addition, as recipes like QLY may relate to very complicated pathologic/physiological process, up to now there has been no suitable method for analyzing the relationships of so numerous components and complicated processes.

Another basic aspect is the determination of synergy effect of medicine combinations.

Currently, it is a new approach of disease treatment to develop medicine composition having synergistic effect. By "synergistic effect" we mean the phenomenon that the effect of two medicine components administrated together is greater than the sum of the effects of the two components administrated respectively. In allowed Chinese Patent Application CN200610114226.X, which was filed by the assignee of the present application and which is incorporated in full text herewith by reference, a medicine composition having synergistic effect was disclosed. However, in the field of medicine development, determination of extent of synergistic effect between medicine components is basically achieved by experiments in vivo and/or in vitro; due to the huge number of possible combinations and complexity of experiment process, high cost and long time were needed for such experiments.

The above are merely two examples of determination of medicine action, which is definitely not limited to the two examples. For example, it also include determing side-effects and etc. of medicines.

Moreover, although the above examples relate to traditional Chinese medicines, the method of the present invention is not limited to any specific type of medicine and is obviously applicable to chemical medicines, biological medicines, composite medicines and so on.

**Summary of the invention**

The present inventors, through extensive research, study and experiment, have established a method for determining medicine action on the basis of gene network.

The present inventors have realized that the features of an action process of a medicine (such as an effect to a disease, a side-effect, synergy effect with another medicine, etc.) are related to the characteristics of distribution of a subset of genes corresponding to the medicine in a gene network. More specifically, such characteristics of distribution include one or more topological attributes of said subset of genes with respect to another subset of genes.

Said another subset of genes can be:

-    a subset of genes corresponding to a bioprocess; or
-    a subset of genes corresponding to another medicine.

According to an embodiment of the invention, in determining the treatment effect of a medicine to a disease, by determining one or more topological attributes of a subset of genes corresponding to the medicine with respect to a subset of genes corresponding to said disease in a gene network, effect of said medicine on said disease can be determined.

According to another aspect of the invention, in determining the synergy effect of a first medicine and a second medicine, by one or more determining topological attributes of a subset of genes corresponding to the first medicine with respect to a subset of genes corresponding to the second medicine in a gene network, the synergy effect can be determined.

According to an aspect of the invention, there is provided a method for determining a medicine action on the basis of a gene network, characterized by comprising:

determining at least one network topological attribute of a first subset of genes/gene products with respect to a second subset of genes/gene products in a gene network,
where
said first subset of genes/gene products includes genes/gene products relating to a first medicine,
said second subset of genes/gene products include genes/gene products relating to at least one of the following:

a second medicine, and
a bioprocess.

According to an aspect of the present invention, for a bioprocess of a disease under consideration, the genes and/or gene products relating to an action of a medicine and the genes and/or gene products relating to said bioprocess are mapped to a gene network, so that extent of action of the medicine on the bioprocess is quantitively determined quickly and accurately at low cost. The method of the invention is suitable for large scale and high efficiency screening and determination of medicine actions.

According to an embodiment of the present invention, a method is provided for determining synergistic effect of a medicine combination including two medicines relating to a disease on the basis of a gene network and medicine-effective genes, comprising:

determining a synergy factor ($ST_{1,2}$) of said two medicines;
determining a medicine similarity factor ($AS_{1,2}$) of said two medicines; and
determining extent of synergistic effect of said two medicines according to a product of said synergy factor and said similarity factor.

In an aspect of the present invention, a method is provided for constructing a disease-related gene network.

In a further aspect of the present invention, extent of synergy effect of two medicines on a given disease is quantitively determined in the context of a gene network relating to said disease by mapping effective genes and/or gene products relating to each of the two medinces to said gene network.

The advantages of the present invention include:

1) the method of the present invention can be implemented on a computer operating Perl; and,
2) the present invention provides an effective approach for pre-clinic determination of extent of synergy of medicine combinations.

The present invention has good prospect for applications in medicine developments. On the one hand it is applicable in quantitive determination of the extent of effect of a medicine on a give disease-related bioprocess (Network-based Assessment for Drug Action), and on the other hand it is applicable in quantitive determination of extent of synergy effect of two medicines on a given disease (Network-based Identification of Multicomponent Synergy). The present invention provides a strong and inexpensive tool for pre-clinic screening and determination of medicine actions and/or synergy effects of medicine combinations.

**Brief description of the drawings**

Figs. 1a-1e show experiment data of each of five pairs of medicines of sinomenine and matrine (SM), sinomenine and honokiol (SH), sinomenine and luteolin (SL), sinomenine and quercetin (SQ), sinomenine and paeoniflorin (SP); and Fig. 1f shows scores of maximum inhibition ratio (MIIR) indicating extent of synergy.

Figs. 2a-2b are diagrams for illustrating robustness of the method of according to embodiments of the present invention with respect to medicine-related gene collections and gene networks.

Fig. 3 is a flowchart showing the method according to an embodiment of the present invention for determining extent of medicine effect based an angiogenesis gene network.

Figs. 4a-4i show experimental results, obtained by the present inventors, of inhibition by matrine, quercetin, emodin, evodiamine, genistein, and aconitine respectively on endothelial cell migration.

**Detailed description**

Defination of some terms relating to the present invention:

1. a "medicine" can be either a single component medicine (such as a traditional Chinese medicine component, a chemical, a biopharmaceutical, etc.) or a composite component medicine (such as a traditional Chinese medicine, a traditional Chinese medicine ingredient, Chinese medicine effective component group, compound group, etc.)

2. "gene network" refers to a network formed by genes or gene products (including biomolecules like protein, product of metabolism, and etc.) through interaction; a "gene network" can be a gene regulatory network, a protein-protein interaction network, signal transduction pathway network, a metabolic network, OR etc; it can also be a network formed by a combination of two or more of these networks.

3. "bioprocess" refers to a pathologic process, a physiological process, a pharmacological process, or a toxic/side-effect process, or other important biological process, or a combined process of a plurality of these processes.

4. "gene product" refers to RNA, protein, product of metabolism, or etc.

Principally, a method of network-based identification for drug action according to an embodiment of the present invention is based on two reasonable considerations. First, for an process of a medicine action and another process (which for determination of medicine action should be a bioprocess such as pathologic/physiological process concerned, while which for determination of synergy effect of a medicine combination should be a process of action of the other medicine(s) in the combination), the subsets of genes/gene products corresponding to these two processes respectively should not be too far away from each other in a gene network concerned, for otherwise the interaction between the two processes must be weak and could not lead to a treatment effect/synergy effect. Second, the extent of a treatment effect or a synergy effect of a medicine(s) is closely related to the importance of corresponding genes/gene products in the gene network, that is, the more important of the genes/gene products relating to a medicine action are in their network, the more remarkable the corresponding effect of the medicine is. The importance of a gene/gene product is measured by the centerness of its corresponding node in the gene network. Based on the above considerations, the present inventors have developed a method for measuring medicine action and determining treatment and/or synergy effect of a medicine(s) based on network topology.

"subset of genes/gene products relating to a bioprocess": a subset of genes/gene products relating to a bioprocess can be obtained from public database, such as Gene Ontology (GO) (http://www.geneontology.org/), or from biological experiments and/or bioinformatical computations.

According to the definition by GO, a "biological process" refers to an ordered assembly of one or more molecular functions. GO has been widely used in life science, particularly bioinformatics, and has established associations among concepts in the fields of biology and united fundamental knowledge, and is regarded as a unification tool of biology. GO includes three relatively independent sections: bioprocess, molecular function and cell component. The three sections completely describe the biological features of gene products, and bioprocess is its primary section.

For example, one can first obtain the subsets of genes/gene products corresponding to endothelial cell migration from biological experiments or database like GO (http://www.geneontology.org/), and obtain subsets of genes/gene products which a candidate medicine acts on under various conditions by literature collection and literature mining on such as CNKI and PubMed, docking, and biological experiments, and etc.

Determination of gene subset relating to a bioprocess will be further explained below with respect to specific examples.

"genes/gene products relating to a medicine" and "subset of genes/gene products relating to a medicine": determination (collection) of genes/gene products relating to a medicine is a work to be carried out prior to the implementation of the method according to the present invention. Specifically, it can be carried out by reading literatures on medicine research and collecting information on genes/gene products relating to a medicine action. Relevant literatures can be downloaded from sources like PubMed (http://www.ncbi.nlm.nih.gov/sites/entrez?db=PubMed) or CNKI (http://www.cnki.net/).

In this way, for each medicine, a group of corresponding genes can be obtained, which is a "subset of genes/gene products relating to a medicine". Determination of subset of genes/gene products relating to a medicine will be further explained later in this specification with respect to specific embodiments.

Since the method of the present invention has good robustness, its requirements on the accuracy of determination of medicine-related genes or gene products and/or accuracy of determination of bioprocess-related genes or gene products are not high. This is a remarkable advantage of the method of the present invention.

For a medicine with composite components, a gene/gene product is determined as belonging to a subset of genes/gene products relating to the medicine whenever the gene/gene product is related to one of the composite components.

"gene network"

An important aspect of the present invention is the determination of the attributes of the topological relationship of two (or more) subsets of genes/gene products in a gene network. The "gene network" can be (but is not limited to) one of the following networks:

- a specially constructed gene network, such as a gene network especially constructed with regard to a specific disease or pathologic/physiological/pharmacological/toxic/side-effect process or etc.;
- all network relationships existing in a public database of protein-protein interactions (such as HPRD), i.e., HPRD global network;
- all network relationships existing in a public database of pathways (such as KEGG), i.e., KEGG global network;
- and etc.

The above-mentioned gene network with regard to a specific disease can be constructed using, for example, LMMA-BioNet biological network construction software co-developed by the present inventors (see Shao LI, Ningbo Zhang, Lijiang WU, LMMA-BioNet Biological Network Construction Software, Chinese Software Copyright Reg. No. 2008SRBJ0202; Reference: Li S, Wu LJ, Zhang ZQ. Constructing biological networks through combined literature mining and microarray analysis: a LMMA approach. Bioinformatics 2006,22: 2143-2150), which includes: downloading abstracts of related literatures containing the disease in their keywords from PubMed; finding genes contained in the downloaded abstracts by matching with dictionary of human genes downloaded from HUGO website (http://www.genenames.org/); and, searching for relationships between two genes in the databases of KEGG (http://www.genome.jp/kegg/) and HPRD (http://www.hprd.org/); determining that an interaction exists between two genes when a relationship is found existing between the two genes. Relationship of functional interaction or physical interaction between two genes can also be realized using one of a plurality of other approaches, such as "concurring", "co-expression of genes", "gene co-regulation", and etc. With the above approach, construction of disease-related gene network can be carried out by a machine (computer); it is not a necessary part of the method of network-based identification for drug action and/or synergy effect of medicine combination according to the present invention; rather, it can be an optional part of the method of the present invention; that is, construction of disease-related gene network can be either a preparative work of the method of the present invention or a part of the work carried out by the method of the invention.

It is shown by the experiments and computations carried out by the present inventors that the final results are not substantially affected by the use of different gene network among the above-mentioned three networks or by adding or deleting edges in the used network, indicating that the method of the invention has very good robustness, which is a remarkable advantage of the present invention.

Example of construction of a disease-related gene network

As an exemplary implementation, the inventors made two independent text files to store the gene relationships in HPRD (http://www.hprd.org/) and KEGG (http://www.genome.jp/kegg/) respectively, with each line in the files representing one known gene relationship. For a pair of genes of interest, matching can be carried out in each of the two files respectively; and, whenever it is found that the two genes appear in one same line in any of the two files, it is determined that an interaction relationship exists between the two genes, i.e., the two genes have a neighboring relationship in their gene network.

As an equivalent but possibly more efficient approach, however, the matching search was carried out in the other direction, i.e., for each pair of genes in HPRD and KEGG databases, it was determined whether both of the genes in the pair appeared in an established subset of disease-related genes/gene products, thus constructing a disease-related gene network.

In an alternative embodiment, the global gene network of HPRD and/or KEGG database can be used as the gene network for implementing the method of the present invention. The effects of the present invention can be realized using different gene networks (as will be explained later).

Embodiment 1: determining synergy effect of a medicine combination

According to an embodiment of the present invention, synergy factor $ST_{1,2}$ of a medicine combination is determined as:

$$ST_{1,2} = \frac{1}{2} \times \left( \frac{\sum_i IP_1(i) \times \exp\left(-\min(d_{i,j})\right)}{\sum_i IP_1(i)} + \frac{\sum_j IP_2(j) \times \exp\left(-\min(d_{j,i})\right)}{\sum_j IP_2(j)} \right) \quad \ldots\ldots\ldots\ldots\text{Formula} \quad (1)$$

where:

-$IP_1(i)$ and $IP_1(j)$ are factors of node importance of network nodes i and j respectively. According to an embodiment of the present invention, factor of node importance $IP$ of a node is determined by using principal components analysis (PCA) to synthetize a NodeRank centerness, a Betweenness centerness, and/or a Closeness ceneterness of the node. Detailed description of use of principal components analysis (PCA), NodeRank centerness, Betweenness centerness, Closeness ceneterness and their determination will be given later in the present specification;

$d_{i,j}$ is the shortest network distance/path from node $i$ to node $j$, and $d_{j,i}$ is the shortest network distance/path from node $j$ to node $i$. Calculation of the shortest network distance will be explained later in the present specification.

According to a further embodiment of the present invention, optionally, medicine similarity and medicine similarity coefficient ($AS_{1,2}$) are introduced in addition to disease-related gene network topological information; the medicine similarity coefficient ($AS_{1,2}$) is used to weight the synergy factor obtained. A basic idea of medicine similarity is that the greater the similarity between diseases treated by two medicines, the greater the possibility that the two medicines have synergy effect when they are used to treat a relevant disease. The relation of correspondance of a medicine and relevant diseases can be retrieved, according to genes, from data in OMIM (Online Mendelian Inheritance in Man, www.ncbi.nlm.nih.gov/omim). Specifically, as long as one gene among the genes corresponding to a medicine is within a set of genes that lead to a disease, we will determine that a relation of correspondance exists between this medicine and this disease. In this way, a first medicine corresponds to a first group of diseases, and a second medicine corresponds to a second group of diseases, and we obtain

$$AS_{1,2} = \frac{\sum_{i,j} P_{i,j}}{N},$$

where $P_{i,j}$ is the similarity factor between diease i in the first group of diseases and disease j in the second group of diseases, and N is for all pairs of diseases between the the first and second groups of diseases.

A method for determinining $P_{i,j}$ was provided by Van Driel, M.A., et al. in "A Text-Mining Analysis of the Human Phenome", Eur. J. Hum. Genet. 2006, 14, 535-542, which included mapping each syndrome item in OMIM database to a set of (0,1) vectors in accordance with standard vocabulary. Thus, two given syndromes, in accordance with their description in words in OMIM database, can be mapped to two sets of (0,1) vectors $\overline{v1}$ and $\overline{v2}$ of standard vocabulary.

Then, the $P_{i,j}$ value is determined as the <u>cosine</u> value cos θ of the angle between the two vectors $\cos\theta = \dfrac{\overline{v1}\cdot\overline{v2}}{|\overline{v1}||\overline{v2}|}$.

Using this method, the present inventors had determined a $AS_{1,2}$ value of 0.17075 for sinomenine + matrine, a $AS_{1,2}$ value of 0.15897 for sinomenine + honokiol, and a $AS_{1,2}$ value of 0.17050 for sinomenine + luteolin.

From the above, a formula for synergistic effect scoring of a method of network-based identification of multicomponent synergy according to the present invention is:

$$S_{1,2} = ST_{1,2} \times AS_{1,2}$$

Then, calculation of network shortest distances (paths) is performed. Floyd Algorithm, which is known as the most effective way for doing this calculation, is used in a preferred embodiment of the present invention. Other algorithm for calculating network shortest distances, however, can be used. All such alternative embodiments are within the scope of the present invention.

Then, node importance is determined. In an embodiment of the present invention, a value of importance factor IP of each node is determined by performing principal components analysis (PCA) at lease one of a NodeRank value, a Betweenness value, and a Closeness value of each node (definitions and algorithms of these values are described later in the present specification); such principal components analysis (PCA) will be described later in the specification, wherein minimum value normalization is performed on any of the three values used.

Calculation of synergy factor is performed in accordance with Formula (1) given above.

Then, a ranking is made for the results based on the above synergy factor. score and ranking of a medicine combination indicates the extent of synergy in treating the disease concerned.

Calculation of matrix of shortest distances/paths in accordance with the preferred embodiment of the present invention

Use of Floyd Algorithm

In Floyd Algorithm, adjacency matrix A=[a(i,j)]n×n of a graph is recursively updated up to n times; that is, from matrix D(0)=A, matrix D(1) is constructed in accordance to a formula; then, D(2) is constructed from D(1) using the same formula; ......; and matrix D(n) is constructed from D(n-1) using the same formula. The element of the i-th row and the j-th column of matrix D(n) is the length of the shortest path from the i-th vertex to the j-th vortex, and D(n) is called the path matrix of the graph. Also, a successor node matrix is introduced to record the shortest path of two points.

The above-mention matrix series can be obtained by iterative method; specifically:

D(i,j):dij(k);

Path(i,j): corresponding to successor point of i on path of d(i,j)(k), with a final value of a successor point of i on the shortest path from i to j;

inputting weighted adjacency matrix A=[a(i,j)]n×n;

1) initiating values

for all i,j,d(i,j)=a(I,j); when a(i,j)=infinite, path(i,j)=0, otherwise path(i,j)=j; k=I;

2) updating d(i,j), path(i,j)

for all i,j, if d(i,k)+d(k,j)>=d(i,j), go to 3); otherwise d(i,j)=d(i,k)+d(k,j), path(i,j)=path(i,k), k=k+1, continuing with 3);

3) repeating 2) until k=n+1.

About PCA method and related parameters:

NodeRank:

NodeRank is an eigenvector corresponding to the maxiumu eigenvalue of network-associated matrix. NodeRank value of node A can be determined by formula:

$$P(A) = \frac{1-d}{N} + d \sum_{v \in L_v} \frac{P(v)}{N_v}$$

where N is the number of all nodes in the network, d is an attenuation factor and is usually set at 0.85, which indicates uncertainty of edges in the network, $L_v$ is the set of nodes that directly connect node A, and $N_v$ is the number of edges of node $v$.

Betweenness:

Betweenness of a node indicates the number of the shortest paths between all pairs of nodes which go through said node. Betweenness of a node provides a very good description of the flow rate which the node possibly undertakes. The greater the Betweenness of a node is, the more data packs that flow through the node, indicating that the node is more likely to be jammed and becomes a bottleneck in the network.
Denoting the number of the shortest paths between any two nodes s and d of a graph as σsd and denoting the number of the paths in these shortest paths passing through node w as σsd (w), the proportion of the number of paths in these shortest paths passing through node w to the number of shortest paths between nodes s and d being $\frac{\sigma_{sd}(w)}{\sigma_{sd}}$,

then Betweenness of node w is defined as:

$$C_B(w) = \sum_{s \in V} \sum_{d \neq s \in V} \frac{\sigma_{sd}(w)}{\sigma_{sd}}$$

Closeness:

Closeness is a measure of importance of a node. Closeness of a node $v$ is defined as the inverse of the sum of the shortest paths from the node to each of all other nodes in the network:

$$C(v) = \frac{1}{\sum_{t \in V} d_{v,t}}$$

where $V$ is the set of nodes in the network that communicate to node $v$, and $d_{v,t}$ is the shortest path from node $v$ to node $t$.

Principal Components Analysis (PCA):

An practical example of application of PCA in the present invention is given below.

Example 1-1:

The inventors used the method of network-based identification for drug/medicine action (also called "NIDA method" ) to select medicine combinations for anti-angiogenesis. 63 candidate medicines were chosen, including 14 medicines of single component and 51 medicines of composite components, which were:

5-fluorouracil, Rapamycin, Vinblastine, Camptothecin, Baicalein, Emodin, Genistein, Honokiol, Indirubin, Luteolin, Matrine, Sinomenine, Paeoniflorin, Quercetin, and

white atractylodes rhizome, rhizoma atractylodis, radix paeoniae rubrathe (unpeeled) root of common peony, monkshood, Ligusticum wallichii, the root bark of the peony tree, the root of red-rooted salvia, Fructus Ziziphi Jujubae, cortex lycii radicis, Rheum officinale, catechu, the root of fangji (Stephania tetrandra), Poria cocos, monkshood, liquorice, the root of kudzu vine, cassia twig, Polygonum multiflorum, flowers carthami, golden cypress, the rhizome of Chinese goldthread, Platycodon grandiflorum, Fructus Tribuli, honeysuckle, Forsythia suspensa Vahl, black false hellebore, Akebia quinata Decne, fructus arctii, the root of bidentate achyranthes, Angelica decursiva, ginseng, pseudo-ginseng, subprostrate sophora, Rhizoma Dioscoreae, maythorn, fructus corni, Acorus gramineus Soland, radices trichosanthis, rhizoma gastrodiae, rhizoma smilacis glabrae, radix clematidis, the fruit of Chinese magnoliavine, evodia fruit, realgar, polygala root, the rhizome of oriental water plantain, fructus aurantii, Cape jasmine, and grifola.

The 63 candidate medicines gave 63*(63-1)/2=1953 medicine combinations.

Among these combinations, two had been known as medicine combinations having anti-angiogenesis synergistic effect: 5-fluorouracil+ Vinblastine, and 5-fluorouracil+ Rapamycin. In predications by NIDA method for all the 1953 combinations, these two medicine combinations ranked among the top three in the ranking of synergistic effect (see Table 1), no matter which of the three gene networks described above was used. These results showed that the NIDA method of the present invention was effective in determining medicine combinations with synergistic effect.

Table 1. NIDA synergistic effect ranking of 5-fluorouracil+ Vinblastine and 5-fluorouracil+ Rapamycin in 1953 medicine combinations

| gene network | | Synergistic effect ranking of 5-fluorouracil medicine combinations by NIDA method | |
| --- | --- | --- | --- |
| | | 5-fluorouracil +Vinblastine | 5-fluorouracil +Rapamycin |
| Disease-specific network | Angiogenesis network | 2 | 3 |
| Global network | HPRD global network | 1 | 2 |
| | KEGG global network | 2 | 3 |

Cell proliferation verification on results of NIDA method

Using NIDA method of the present invention, the present inventors determined scores of synergistic effect of five medicine combinations: sinomenine+luteolin, sinomenine+quercetin, sinomenine+honokiol, sinomenine+matrine, and sinomenine+paeoniflorin. Further, the present inventors verified the results of the scores of NIDA method of the present invention by cell proliferation verification experiments.

Cell proliferation verification experiments: human umbilical vein endothelial cells (HUVEC) were purchased from Cascade Biologics. Culture; M200 culture medium containing 2% low density of fetal bovine serum and 5ng/ml of bFGF was used in cell culture; incubation of culture was carried out under 37 °C and 5% $CO_2$ environment; passage culture was carried out at 1:2 passage ratio using pancreatin/EDTA as assimilation liquid. HUVECs of the 3th-6th generations were used in all the experiments. Small molecule standard substances of sinomenine, luteolin, quercetin, honokiol, matrine, and paeoniflorin were purchased from National Institute for the Control of Pharmaceutical and Biological Products.

Density, dosage, and combination ratio of each small molecule and medicine combination were designed on the basis of the description of relevant references. DMSO hydrotropy was used for sinomenine, luteolin, quercetin, honokiol, and paeoniflorin, while matrine could be directly dissolved in culture medium. Each medicine was preserved under -20 °C environment as a mother liquor with a final concentration of 50mM. Extent of cell proliferation was evaluated using the Cell Counting Kit CCK-8 developed by DOJINDO Laboratories. Specific experiment steps included performing 24 hour cell proliferation, adding two medicines separately and in combination into 96 orifices, adding 10 microlitres of CCK-8 reagent to each of the orifices 48 hours after pharmacologic intervention, and then incubating the 96 orifices for 4 hours under 37 °C followed by OD value measurements. Each group of experiments was repeated for three times.

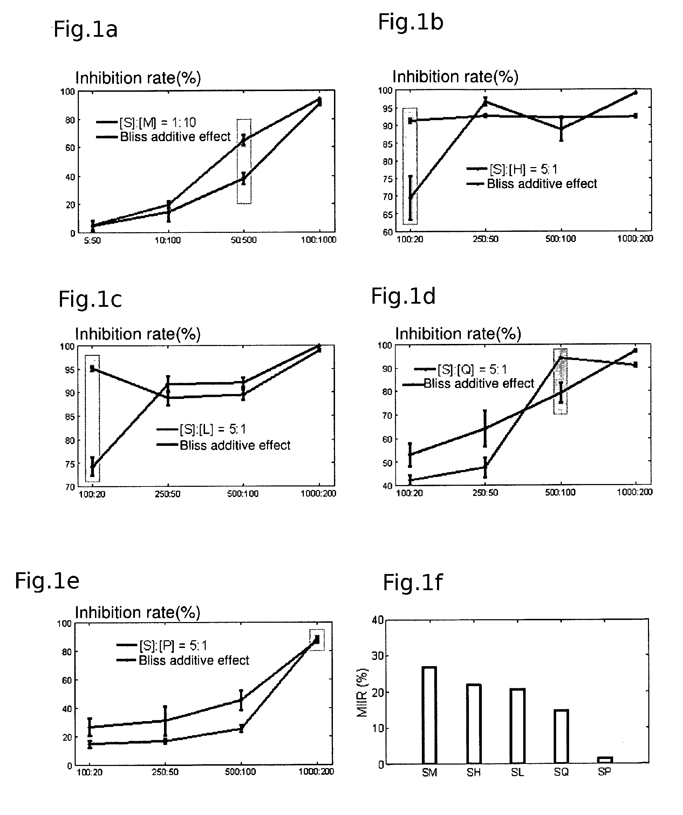

Figs. 1a-1e illustrate experiment data for sinomenine and matrine (SM), sinomenine and honokiol (SH), sinomenine and luteolin (SL), sinomenine and quercetin (SQ), and sinomenine and paeoniflorin (SP), respectively, where each gray line indicates inhibition ratio of the medicine combination concerned to HUVEC cell proliferation (curve of dosage dependency) and each black line indicates corresponding curve of Bliss additive effect.

Comments: by this study, the inventors considered each low-dosage combination with an inhibition ratio greater than 70% as an effective combination, and the inventors evaluated synergistic effect using dosages and combination ratios, Bliss additive effect model, and maximum inhibition ratio reported in pharmacal literatures. Whether a medicine combination had synergistic effect was determined using Bliss additive effect model. To further evaluate intensity of synergistic effect, the inventors used formula $MIIR=max(IR_{syn} - IR_{add})$ to calculate maximum increased inhibition rate, MIIR, with $IR_{syn}$ and $IR_{add}$ respectively representing measured inhibition ratio and added inhibition ratio of the medicine combination concerned.

Fig. 1f shows score of maximum increased inhibition rate (MIIR) of each of the five medicine pairs, which indicates extent of synergistic effect, wherein maximum synergistic effect ratio of each medicine combination is indicated by a histogram. It can be seen from Fig. 1f that MIIR score of each of the five medicine pairs was in consistent with the ranking of NIDA predication.

Result analysis: by comparing results of cell experiments and those of NIDA prediction (Table 2), it was shown that ranking obtained from results of cell experiments were fully consistent with results of synergistic effect intensity determined by NIDA prediction based on gene network, so the reliability of NIDA method of the present invention was verified. Also, using NIDA method of the present invention, the inventors found three sinomenine combinations each having relatively strong synergistic effect on anti-angiogenesis, which were: sinomenine+matrine (MIIR=26.83%), sinomenine and honokiol (MIIR+22%), and sinomenine and luteolin (MIIR=21 %). The medicine combination of sinomenine and paeoniflorin, which ranked last in NIDA ranking and cell experiment verification, yielded an MIIR of only 1.86%, indicating that it had little synergistic effect.

The present inventors further investigated the robustness of NIDA method of the present invention with respect to the gene network used. For this, the present inventors made predictions using each of the three networks respectively, which resulted in basically the same outcomes, as showed in Table 3. In terms of prediction accuracy, results obtained by using disease-related gene network (angiogenesis network) and HPRD global network respectively were better, while result obtained by using KEGG global network was relatively inferior.

Table 2: Prediction scores of synergistic effect of medicine combinations including Sinomenine by NIDA Method based on angiogenesis network

| Ranking | Medicine combined with Sinomenine | Synergistic effect score by NIDA prediction | | Cell experiment MIIR value |
|---|---|---|---|---|
| | | NodeRank, Betweenness, Closeness | Betweenness, Closeness | |
| 1 | Matrine | 0.10923 | 0.117280 | 26.83% |
| 2 | Honokiol | 0.10142 | 0.112270 | 22% |
| 3 | Luteolin | 0.10007 | 0.105980 | 21% |
| 4 | Quercetin | 0.09835 | 0.104840 | 15% |
| 5 | Paeoniflorin | 0.082148 | 0.084320 | 1.86% |

Table 3:Prediction scores of synergistic effect of medicine combinations including Sinomenine by NIDA Method based on three networks

| Medicine combined with Sinomenine | NIDA synergy effect ranking | | |
|---|---|---|---|
| | angiogenesi s network | HPRD global network | KEGG global network |
| Matrine | 1 | 2 | 1 |
| Honokiol | 2 | 1 | 3 |
| Luteolin | 3 | 3 | 4 |
| Quercetin | 4 | 4 | 2 |
| Paeoniflorin | 5 | 5 | 5 |

Steps of an embodiment of NIDA method:

1. In an embodiment, where angiogenesis network was used, computation of NodeRank, Betweenness, and Closeness in NIDA method and PCA were carried out as follow:

(1) The angiogenesis network that the present inventor constructed in a method of construction of disease-related network as described above was a connected sub-network containing 1893 gene nodes and 7598 edges. Data of the angiogenesis network are available and downloadable at:

http://bioinfo.au.tsinghua.edu.cn/member/nzhang/download/angiogenesis.txt wherein all genes are indicated by Entrez GeneID of NCBI.

(2) NodeRank, Betweenness, and Closeness of each of these nodes were calculated in accordance to the above definitions of these parameters. To ensure comparability among the data, they were normalized by dividing their respective minimum. Thus, a 1893*3 matrix (denoted as Matrix A) was obtained. Matrix A is given in the part of this specification subtitled " Part I: Noderank-Betweenness-Closeness values of Angiogenesis Network", where the three values contained in the i-th pair of parentheses are the NodeRank, Betweenness, and Closeness values respectively of the i-th gene listed on webpage:
http://bioinfo.au.tsinghua.edu.cn/member/nzhang/download/angiogenesis.txt

(3) PCA (principal components analysis) on the three 1893* 1 vectors, which were constructed by the three columns of Matrix A respectively, was conducted in MATLAB. Denoting the final node importance factor vector as IP, then the commands in MATLAB were:

[COEFF, SCORE] = princomp(A); IP = -A*COEFF(:,1)
Of course, we could also choose only two of the three vectors for PCA to obtain the IP value of the node. When only Betweenness and Closeness were chosen and the then obtained 1893*2 matrix was referred to as C, the corresponding commands in MATLAB were:
[COEFF, SCORE] = princomp(C); IP = -A*COEFF(:,1)
In embodiments of the present invention, the present inventors applied NIDA method to the combinations of Sinomenine-Matrine, Sinomenine-Honokiol and/or Sinomenine-Luteolin, respectively, in the following way:

(1) First, IP value of a gene relating to a medicine was the IP value of the corresponding node (gene) in the disease-related network. IP values corresponding to respective genes of sinomenine, matrine, honokiol, and luteolin respectively are given in the parts of this specification subtitled "Part II" to "Part V" respectively.

(2) Then, on the basis of matrix of shortest paths of the angiogenesis network, a matrix of shortest paths among genes for each of the medicine pairs Sinomenine-Matrine, Sinomenine-Honokiol and Sinomenine-Luteolin was obtained, as given in the parts of this specification subtitled "Part VI" to "Part VIII" respectively, where the lines in each of the matrix corresponds to the genes of sinomenine and columns corresponds to the genes of the other medicine. From this network topology information, the ST value of each of the pair of medicines was obtained (as 0.63971 for Sinomenine-Matrine, 0.63800 for Sinomenine-Honokiol, and 0.58692 for Sinomenine-Luteolin).

(3) ST value of each of the pairs of medicines was weighted by corresponding AS value (Sinomenine-Matrine: 0.17075, Sinomenine-Honokiol: 0.15897, Sinomenine-Luteolin: 0.17050) to obtain a final value of synergy factor S (as 0.10923 for Sinomenine-Matrine, 0.10142 for Sinomenine-Honokiol, and 0.10007 for Sinomenine-Luteolin).

(4) These pairs of medicines were ranked according to the above results of extent of synergy of each of them.

On robustness of NIDA method

1. Robustness with respect to random addition/reduction of medicine-effective genes

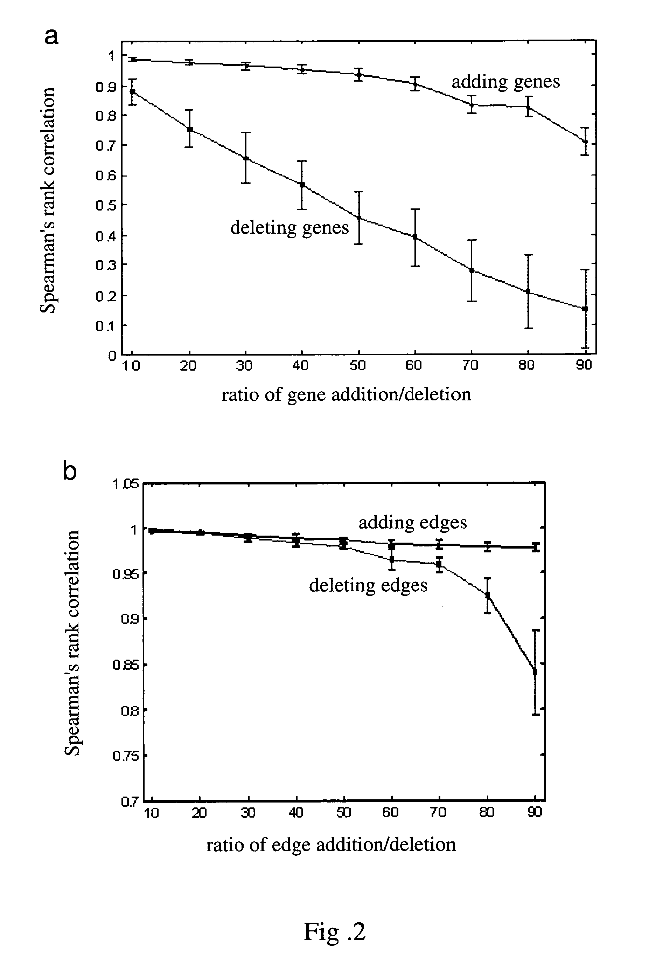

In collecting medicine-effective genes by reviewing literatures, neglects and/or mistakes in the references can hardly be avoided. The present inventors evaluated the robustness of NIDA method by randomly increasing/decreasing the genes relating to medicines, and described the robustness evaluation by comparing Spearman rank pertinence before and after permutation, and obtained the results as shown in Fig. 2. As the results shown in Fig. 2 indicated, effect of random addition of genes on result of NIDA method was not obvious, while effect of random deletion of genes on result of NIDA method was relatively remarkable; however, even when the number of genes was deleted by 50%, the pertinence of NIDA result to NIDA result without gene deletion still reached 50%. The above experiment outcomes indicated that NIDA method of the present invention had strong robustness to collection of medicine-effective genes.

2. Robustness with respect to random increase/decrease of gene network

Construction of gene network may be inaccurate and/or incomplete, and the ways of construction may be different, leading to difference in the gene networks concerned. To evaluate the effects of such differences on the results of the NIDA method of the invention, the present inventors conducted random increase and decrease of the gene network to measure the robustness of the NIDA method of the invention to gene network., wherein the robustness evaluation was measured by comparing Spearman rank pertinence before and after the increase/decrease of the network. The result was as shown in Fig. 2b, which indicates that neither random increase of edges in the network nor random decrease of edges in the network had obvious effect on the results obtained by the method of the invention. This indicated that the NIDA method of the invention had good robustness with respect to the gene network.

Part I: Noderank-Betweenness-Closeness values of Angiogenesis Network (Matrix A)

(28.304,29.846,2.7015);(1.2325,1,1.7449);(24.564,22.378,2.541);(1.2257,1,1.7447);(2.0985,1.0421,1.9812); (1.826,1.0112,2.0015); (2.2416,1,1.68511);(2.0573,1.4359,2.1775);(4.6517,3.0379,1.7682);(3.4778,1.1472,2.3706); (3.9337,1.1581,1.9943);(38.952,33.064, 2.8982);(3.3814,2.5698,1.9309);(1.5289,1,1.9501);(1.0724,1,1.9262); (5.4903,3.7287,2.0068);(9.1525,3.625,2.4496);(15.469,5.675 7,2.5692);(4.6133,2.6714,2.068);(12.307,4.2026, 2.3747);(3.1998,1.1501,1.8779);(6.4805,3.7035,2.1668);(3.0421,1.2686,2.124);(8. 0527,1.6802,1.9328);(5.3362, 2.4403,2.0612);(5.1311,4.0996,1.9438);(4.5309,1.1234,2.3687);(3.8859,1.1694,1.7156);(1.4309,1,1.6 823); (4.1645,1.3791,1.7071);(2.2703,1.3173,2.0826);(1.3369,1,1.7451);(1.9414,1,1.4263);(1.1335,1,2.1322); (14.095,6.4194,2.378) ; (3.7074,5.548,2.193); 15.486,7.3526,2.6097); (1.0464,1,2.1145); (6.0541,1.2202,1.8854); (2.7165,1.2,1.8628);(6.0701,2.9979,1.429 7);(7.0989,2.8667,2.2514);(1.3127,1,1.6808);(1.6399,1.0176,1.7724); (7.0334,2.7518,2.236);(3.2205,1.2792,1.9291);(5.8846,4.1141 ,2.2384);(6.1965,2.7663,2.3947);(6.9995,4.4391, 2.4193);(17.786,9.0719,2.5815);(1.1212,1,1.9316);(6.2869,2.4204,1.9626);(2.1177 ,1.0803,2.1609);(110.208, 3.6215,2.4799);(3.6106,1.1189,2.1747);(1.2626,1,1.7697);(8.4619,3.3604,2.4358);(1.4636,1,1.6027);(5.54 71, 4.131,2.3624); (26.389,16.534,2.7565); (2.0032,1.0023,1.9368); (2.2835,1.1076,1.9794); (3.3908,2.309,2.1228); (5.0891,2.0201,2. 1728);(1.2489,1,1.7544);(8.3371,6.17,2.2873);(1.4766,1,1.6405);(6.9743,1.6134,2.1103);(3.6729, 1.2107,2.1388);(4.0415,1.3411,2. 3432);(3.738,1.0147,2.3162);(5.4189,1.4332,2.3259);(5.9447,3.0432,2.3863); (5.2243,3.0499,2.3277);(1.7853,1,1.6639);(1.9881,1, 1.2274);(1.3043,1,1.6992);(1.6554,1,1.5048);(2.7575,1.4891, 2.2036);(3.2597,1.3797,1.9463);(4.3039,2.9837,2.2072);(2.0251,1.40 49,2.02);(4.9476,1.8792,2.1498);(3.3176, 1.5054,1.989);(1.3064,1,1.6471);(1.0712,1,2.1151);(12.261,9.3364,2.4158);(2.7549,1.127 6,2.0328);(1.0961,1, 2.0023);(5.4327,3.1294,2.0444);(1.2562,1,1.7747);(5.1927,3.0036,1.6167);(15.259,12.342,2.3241);(1.5148, 1.0 015,1.884);(8.7888,3.2494,2.3981);(4.2793,1.1514,2.1529);(4.1271,2.435,2.0737);(3.121,2.0681,2.2454); (5.0179, 2.8864,2.2301);( 1.291,1,1.6604);(12.273,10.813,2.1331);(1.3765,1,1.6098);(2.7841,1.0205,2.0422); (3.6895,1.5383,2.2457);(1.4859,1,1.6584);(1.89 19,1.0263,2.2511);(1.1883,1,1.8861);(1.6719,1.0024,1.8793); (2.7529,1.1509,1.9721);(1.831,1.0003,1.6927);(5.3596,3.9649,1.641); (2.0986,1.0067,1.9341);(4.4589,2.5125, 1.8868);(2.2027,1.021,2.1557);(1.9325,1,1,4136);(13.408,4.3841,2.3962);(2.2504,1.2352,1. 998);(1.6824,1,1.3849); (1.6388,1.039,2.0159);(2.3948,1.1932,1.5444);(2.4824,1.0425,2.0224);(5.0687,2.9388,2.3962);(3.3464,1,1. 3014); (15.236,9.7939,2.6184);(1.3734,1,1.6477);(3.975,3.57,1.9301);(16.151,6.5691,2.5419);(2.2352,1.1069,2.0575); (20.889,12.8 99,2.7054);(1.604,1,1.4132);(7.461,3.8938,2.1731);(1.0741,1,1.7231);(1.365,1,1.6714);(5.6803,3.3867, 2.1731);(2.8509,1.0225,2.0 195);(4.2876,1.883,2.066);(4.1552,2.2374,2.204);(7.0539,5.7621,2.2205);(4.192,1.6853, 2.2444); (3.9513,1.7254,1.9631); (1.441,1,1 .272); (5.8707,2.234,2.4228); (2.2236,1,1.6877); (4.1054,3.3029,2.2602); (1.9736,1.006,1.9494);(3.6496,1.0249,2.1841);(2.2352,1.04 3,1.6316);(6.9391,1.3929,1.7888);(24.989,11.23,2.5846); (4.8474,2.9974,1.4526);(1,1479,1,1.8954);(1.1594,1,1.7783);(3.6496,1.02 49,2.1841);(1.5064,1.006,2.0927);(3.7341, 2.1,1.9232);(1.4769,1,1.557);(2.5605,1.0888,1.9433);(1.3349,1,1.7245);(1.0942,1,2.052 2);(4.1058,1.6685,1.9448); (6.7117,2.4615,2.4201);(8.3156,1.7904,2.2447);(2.4772,1.1507,1.9406);(19.157,22.035,2.5679);(6.5662, 2.4324, 2.2626);(5.032,1.3697,1.4888);(7.0874,1.6887,2.2504);(12.996,10.275,2.4429);(1.8236,1.4653,1.9964);(5.127, 1.983,2.2153 );(1.3884,1,1.6039);(2.7774,1.0793,1.9822);(3.2477,1.5308,2.1204);(1.6611,1,1.4267);(1.4458, 1,2.1791);(1.3522,1,1.6778);(3.2357 ,1.1063,2.2694);(5.5465,2.4101,2.0606);(18.854,4.8555,2.5653);(2.2127,1.3231, 1.7145);(3.4238,1.9992,1.4134);(2.2194,1.2201,1. 7356);(7.8629,4.2964,2.0832);(4.2065,1.8364,2.1181);(4.6977, 1.7286,2.0116);(4.8928,1.5159,2.2595);(6.2976,3.9021,2.1083);(1.7 415,1,1.7059);(1.1244,1,1.755);(3.5176,2.0178, 1.6767);(8.5014,4.7542,2.2261);(1.543,1.0083,1.9641);(3.582,2.292,2.068);(7.7272 ,2.5445,2.3886);(4.5698,1.0707,2.2575);(4.4494,2.0209,2.3141);(3.4908,1.3308,1.8279);(10.592,2.2365,2.3803);(5.0907,2.5392,2. 2626);
(7.4515,4.0803,2.4046);(5.3556,1.4903,2.3583);(1.6666,1.0986,2.0298);(3.0664,1.6008,2.0323);(2.0177,1.6304, 1.9527);(1.5 289,1,1.9501);(1.7622,1.3533,2.1416);(2.0276,1.0213,1.6716);(7.2268,7.4736,2.475);(5.2265,2.4507, 2.0181); (1.2039,1,1.6821); (6. 1809,1.7761,2.4016); (2.0704,1.0428,1.9814); (4.2889,1.4684,1.5924); (1.7948, 1.0074,2.1684); (1.579,1.0433,2.241); (3.1747,2.8064, 1.7143); (3.819,1.2479,1.9817); (6.6823,4.3232,1.6279); (2.2491,1.0098,1.7888); (2.4149,1.1246,2.1385); (1.101,1,1.6975); (2.0046,1. 0231,1.9532); (1.5959,1,1.683); (1.9697, 1.0055,2.1041); (5.3797,1.9163,1.5625); (2.1347,1.0015,1.7633); (3.6376,3.6449,2.2222); (2. 815,1.089,1.9567); (4.1676,1.097,1.6223); (5.018,3.2041,2.3381); (3.1022,1.2583,2.1918); (5.1572,3.174,1.8646); (1.8635,1.0116,1.7 424); (2.5752,1.0594,2.0268); (5.4484,2.6595,2.1825); (1.5158,1.0115,2.0835); (15.115,9.8137,2.1535); (2.2897, 1.0009,1.7823); (4.47 78,1.0079,1.8171); (8.0355,6.4172,2.0279); (1.9094,1.0044,1.7499); (1.6728,1.0002,2.0023); (2.6733,1.0495,1.5687); (1.0804,1,2.019 2); (5.8155,3.189,2.4588); (1.1883,1,1.8861); (1.1286,1,1.8169); (1.3043,1,1.6992); (1.5466,1,1.5366); (2.5622,1.0985,1.8875); (5.7185 , 1.7317,2.202); (1.3938,1,1.728); (5.1396,1.1316,2.2531); (1.7139,1,1.2455); (1.9751,1,1.2412); (6.0998,2.3783,2.3553); (9.133,3.002 8,2.3169); (2.1047,1.0526,1.7937); (3.3663,1.2138,1.9549); (4.9364,2.4265,2.2176); (4.3203,1.4961,2.3291); (6.1006,1.4973,2.1991); (2.6048,1.035,1.5428); (1.3349,1,1.7245); (1.2821,1,1.5982); (3.7968,2.422,2.0087); (2.8212,1,1.7867); (4.7016,2.5161,2.0916); (3.41 55,2.1455,2.1772); (2.5562,1.2639,2.1765); (2.3381,1.2762,2.2633); (2.0621,1,1.6952); (1.0465,1,1.829); (1.2358,1,1.6219); (1.5378,1 , 1.542); (5.0725,1.0134,2.1653); (1.2067,1,1.6953); (4.7123,2.4805,2.34); (1.2664,1,1.7096); (3.1791,1.5948,1.6062); (4.8456,2.1749, 1.9595); (5.6345,4.6418,1.7745); (2.6473,1.243,1.884); (2.3764,1.594,2.0646); (4.8053,1.7683,1.8434); (2.3875,1.0354,1.9888); (6.83 88,1.4614,2.4856); (1.9024,1.028.1.7888); (5.1498,3.4612,2.1207); (5.3436,2.7266,2.2531); (1.5258,l, 1.7123); (12.104.5.4543,2.4275 ); (4.5125,1.8952,2.2926); (10.737,11.167,2.2839); (6.3475,1.8615,2.2877); (2.5564,1.3877,1.8734); (1.8803,1,1.392); (12.052,7.9716, 2.2481); (1.3057,1,1.5807); (2.2552,1.3475,1.776); (6.5076,2.299,1.9208); (2.9794,1.1869,2.0475); (4.0236,2.9974,1.3353); (1.3612,1, 1.4348); (2.0531,1.0112,1.9112); (3.2451,1.3875,2.2182); (1.1063,1,1.8262); (1,134,1,1.7467); (1.7228,1,1836,1.8916); (1.5969,1,2,14 52); (1.9234,1.0608,1.8304); (2.6246,1.9992,2.066); (5.1549,2.0896,2.1905); (3.3784,1.0092,1.8064); (18.997,12.286,2.6633); (2.0243 , 1.0116,1.9988); (12.042,3.992,2.3262); (16.406,8.4075,2.3031); (2.1347,1.0015,1.7633); (4.6741,6.0081,2.0068); (12.394,4.3724,2.3 863); (1.3034,1,1.7989); (1.2495,1,1.6781); (2.2536,1.0344,2.1053); (1.2255,1,1.803); (3.7461,2.035,1.8322); (4.973,4.0354,2.1056); (2 . 4805,1.2319,1.9338); (1.1936,1,1.6872); (2.0623,1.1916,1.8623); (29.049,27.518,2.8158); (3.5526,2.2603,2.1934); (4.7793,1.3758,1. 8861); (8.1677,3.8815,2.336); (3.1713,1.1221,2.2698); (2.4149,1.1246,2.1385); (3.0582,1.2992,1.7061); (2.1204,1.0071,1.6701); (3.62 88,1.8575,2.0663); (1.3281,1,1.5664); (5.7104,1.8418,2.236); (2.8017,1.9992,1.6957); (1.9813,1.5371,1.8454); (1.6902,1,1.1186); (1.2 947,1,1.7188); (22.256,17.571,2.6723); (1.683,1.0385,1.9846); (2.3734,1.0051,1.9451); (1.1335,1,2.1322); (1.5703,1,1.9064); (6.3658, 3.1673,1.7164); (1.0272,1,1.9391); (2.1293,1,1.3017); (2.8856,2.0035,2.07); (1.0328,1,1.8563); (1.2085,1,1.8127); (3.066,1.0003,1.15 57); (3.022,2.1824,1.7125); (2.78,1.3091,2.0878); (3.4529,2.1976,2.1352); (3.3974,1.7947,2.2075); (5.1631,2.9478,1.9383); (1.3239,1, 1.5216); (8.3408,3.4257,2.038); (5.8407,2.9715,2.4069); (3.1497,1.018,2.1443); (3.4803,1.1018,2.1634); (2.3969,1.0945,1.5568); (1.9 011,1.0311,1.7695); (2.0959,1.0082,1.6079); (3.6573,2.1306,2.1328); (1.5591,1.0119,2.0464); (1.9577,1,1.7487); (4.0117,1.9467,2.21 27); (5.0143,2.7821,2.1921); (8.4847,6.0317,1.6982); (2.6683,1.0291,1.9479); (2.2929,1.0933,1.6348); (5.8214,2.8923,2.1841); (5.824 3,2.0875,1.919); (5.2707,3.7734,1.9368); (1.0961,1,2.0023); (1.4934.1,1.4323); (1.9598,1,2.1012); (18.155,8.1451,2.5509); (1.8753,1, 2.1972); (1.7817,1,1.5107); (5.6034,1.7595,1.5247); (1.8017,1.0136,1.7055); (9.1221,5.6783,2.4783); (1.3385,1,1.5988); (1.2626,1,1.7 697); (3.0185,1.0518,1.9534); (6.9501,5.6803,2.3465); (3.1575,1.0216,1.8375); (1.5691,1.0062,1.8809); (2.1741,1.0201,1.8699); (17.3 66,5.7295,2.5785); (3.04,1.0397,2.1615); (1.8188,1.0003,2.2101); (2.7921,1.8991,2.2062); (1.0326,1,1.9964); (2.8746,1.0719,2.0071); (2.5497,1.0665,1.8456); (2.0156,1.2511,2.1696); (10.834,3.601,2.2894); (3.3988,1.2553,2.2626); (4.4499,2.3653,2.1784); (6.4854,1.7 304,2.2957); (1.7984,1.0076,2.1458); (1.7892,1,1.3438); (1.2006,1,1.8835); (2.1347,1.0015,1.7633); (4.0551,1.7287,2.2619); (1.4077, 1,2.0809); (3.7232,2.1421,1.9356); (5.7221,3.5427,1.5552); (1.1136,1,1.9127); (6.1567,3.0398,2.11); (9.3992,5.4395,2.3698); (4.1876, 2.6926,2.0971); (7.6335,3.4533,2.359); (4.5036,1.3335,2.1507); (4.209,2.2738,1.9996); (1.6344,1.0046,1.9319); (5.545,2.7976,1.695); (2.4642,1.1683,2.1391); (4.6282,3.1452,2.4154); (2.3398,1.0823,1.6453); (1.642,1.0006,1.7429); (4.5701,1.3102,2.3151); (3.6407,1.5 59,2.1765); (3.9714,2.2589,2.3996); (2.3604,1.0314,1.5501); (6.9874,3.2887,2.1409); (11.042,8.0621,2.3799); (2.5939,1.0337,1.9718) ; (1.606,1.02,1.9875); (1.4113,1,1.5715); (2.9335,1.9992,1.7188); (1.0772,1,2.0455); (1.3734,1,1.6477); (2.1499,1.008,1.49); (2.4947,1. 0162,2.2701); (4.1828,1.1267,2.1921); (5.1963,1.7992,2.1715); (4.1332,2.2649,2.0087); (2.102,1,1.2431); (6.4136,2.0553,2.344); (8.7 143,4.5835,2.4178); (5.1396,4.7831,2.0881); (3.9049,1.3323,1.9494); (1.5823,1.0023,1.8117); (2.4089,1.0132,2.2004); (3.6496,1.024 9,2.1841); (2.713,1.2021,1.7802); (3.1462,1.27,2.2156); (2.8439,1.1134,2.0522); (4.5627,1.3171,2.0157); (2.7198,1.1892,1.7506); (1.8 385,1.0171,2.0677); (3.2297,1.0001,1.4774); (1.9731,1.0012,1.9867); (1.8051,1.0001,1.479); (8.3441,2.7863,2.2052); (1.2562,1,1.774 7);

(2.6246,1.9992,2.066); (2.0671,1.0126,1.7483); (1.2527,1,1.7225); (3.0727,1.493,2.0814); (5.1041,2.5741,2.1628); (2.1006,1.0716, 2.1258); (5.3748,1.8385,2.2894); (4.6634,1.9992,1.1437); (3.4953,1,1.4762); (9.4314,4.0807,2.3363); (2.401,1.039,1.3314); (3.4257,1. 3906,1.9854); (2.4833,1.0001,1.2554); (7.6461,3.4949,2.0609); (4.8171,2.2516,2.3432); (2.4699,1.0948,1.8217); (1.5071,1,2.1902); (4 . 1869,2.8811,2.082); (1.2828,1,1.6908); (2.4117,1.067,1.7046); (1.9806,1.0552,2.0592); (6.069,5.0018,1.8073); (2.1663,1.0136,1.740 2); (2.9992,1.5685,2.0884); (1.6078,1,1.4615); (2.4455,1.0573,1.8138); (1.8012,1,1.6403); (6.9538,3.0005,2.4318); (3.0383,2.0469,1.9 042); (1.9136,1,2.1425); (3.3322,1.9992,1.4732); (3.5833,1.6214,2.1825); (7.1679,1.9107,2.3066); (1.4588,1,1.412); (5.8057,1.7236,1. 6527); (6.5691,2.1289,2.4726); (4.1057,1.6037,1.8901); (3.0317,1.0417,2.236); (4.4465,4.2433,1.803); (4.269,2.6541,2.1507); (1.3837 ,1,2.1243); (9.5304,3.7026,2.4464); (3.6301,2.001,2.268); (2.8668,1.0445,2.203); (4.3778,1.3646,1.9946); (2.4429,1.3221,2.0754); (1. 2909,1,1.7184); (2.5929,1.135,2.1297); (17.527,9.8297,2.3905); (1.0975,1,2.0542); (5.8505,5.3883,1.8114); (1.1081,1,1.8713); (1.1335 ,1,2.1322); (3.9162,1.3969,2.0592); (1.2672,1,1.7273); (5.1714,4.5231,2.0843); (1.1684,1,1.7846); (2.8687,2.0892,2.1222); (1.1759,1, 1.7764); (18.755,8.0872,2.5967); (4.5081,1.1057,1.9134); (5.4436,3.3236,2.2111); (3.1041,1.6179,2.2863); (2.2257,1.2088,1.7899); (3. 0393,1.0501,1.9269); (3.883,1.5318,2.2127); (4.5834,2.105,2.0869); (3.9275,2.954,2.1258); (1.7159,1.0009,1.873); (1.693,1.0275,1.8 6811); (15.386,6.3955,2.6732); (4.0316,1.6974,2.0598); (1.5724,1.0026,1.8371); (2.2416,1,1.68511); (3.5053,1.484,1.7241); (9.4197,3.8 632,2.3561); (4.3592,1.0504,1.5597); (4.0042,1.5293,1.5789); (4.4465,1.6078,2.3144); (11.737,7.9617,2.3627); (2.3537,1.0122,1.918 8); (4.5891,1.3747,1.5929); (1.1323,1,1.9321); (1.6298,1.0151,2.0162); (43.73,33.349,2.7651); (3.6496,1.0249,2.1841); (1.8698,1,1.16 91); (5.8105,1.8817,2.1285); (3.3624,1.4363,1.8436); (1.8299,1,1.5323); (5.0686,2.1872,2.3041); (3.8418,1.4813,1.9144); (1.1944,1,1. 8589); (3.8363,1.1892,1.8293); (2.7529,1.0541,1.7318); (7.0241,5.4145,2.4275); (4.983,4.077,2.1914); (4.3814,2.0424,2.1027); (6.131 4,1.9749,2.0863); (5.684,3.4185,2.186); (1.5096,1.0048,1.9595); (2.6116,1.0728,2.0708); (1.2083,1,1.7827); (37.297,31.797,2.7686); ( 7.0622,4.2476,2.151); (17.066,5.6075,2.5557); (1.8709,1.0717,1.8746); (17.703,6.4704,2.5705); (2.1436,1.0978,1.9675); (4.9058,4.42 17,2.3443); (1.7703,1,1.9595); (4.4842,1.5641,2.0983); (5.1355,1.4512,2.4042); (14.698,5.6875,2.4209); (4.4178,1.6107,2.0697); (4.6 907,2.3494,2.2701); (5.5259,3.1486,1.9132); (1.2607,1,1.7802); (3.1219,1.l233,1.8402); (3.3055,1.4311,2.1343); (4.213,2.8196,2.295 7); (3.2209,1.0461,2.2017); (1.315,1,1.7532); (27.03,27.96,2.8063); (2.9703,1.2351,2.2497); (3.4223,2.0945,1.8556); (1.4572,1,2.0079 ); (2.6287,1.3593,2.1222); (2.4989,1.1741,1.4916); (1.7635,1.0242,1.9168); (15.391,8.3992,2.4836); (2.2803,1.0211,1.6005); (2.2339, . 0282,1.7618); (2.3561,1.0309,1.6896); (1.1321,1,1.9499); (7.884,6.4156,2.2248); (5.2885,2.5651,2.4653); (3.2447,1.0457,1.5824); (1. 4444,1,1.5307); (1.7177,1,1.5461); (1.8918,1,1.7459); (1.5604,1.0163,1.818); (1.8391,1.0127,2.0751); (1.3734,1,1.6477); (2.4819,1.05 , 1.7506); (12.127,8.1668,2.3724); (2.319,1.0672,2.1334); (4.1738,1.689,2.2444); (3.9184,1.1105,2.2701); (1.8335,1.0161,1.8536); (3.0 161,1.3137,2.0623); (2.6187,1.6347,1.8093); (1.7827,1.0189,1.8522); (16.739,17.072,2.5846); (3.2379,1.0013,1.4007); (2.0077,1.000 4,1.9705); (23.509,30.505,2.7891); (1.7868,1.0072,1.7925); (1.0748,1,1.8732); (21.456,13.027,2.6747); (5.7118,1.7911,2.2873); (9.748 , 4.6972,2.3542); (4.438,4.4096,2.0028); (2.4134,1.8636,2.0812); (18.121,9.0195,2.5176); (1.0505,1,1.9562); (1.267,1,1.7808); (5.2869 , 1.6521,1.4901); (1.7337,1.0032,2.0731); (1.0961,1,2.0023); (6.5076,2.299,1.9208); (6.1412,1.0781,2.1994); (6.4378,2.5419,2.3784); ( 2.2474,1.0802,1.9494); (1.1244,1,1.7684); (7.6329,3.9973,2.1033); (9.4441,3.3854,2.0866); (4.7029,1.8343,2.2528); (13.357,10.043,2 . 6088); (1.1081,1,1.8713); (1.8398,1.3407,2.089); (14.93,9.0865,2.2756); (3.6004,1.0082,1.686); (23.86,20.647,2.723); (3.0199,1.296, 1.5533); (14.131,10.669,2.4803); (4.6755,2.3195,2.2548); (8.0775,6.5547,2.0519); (3.7012,2.4507,2.2484); (1.5837,1.0098,2.0184); (2 . 1156,1.0079,1.9501); (8.7734,3.362,2.2514); (1.7059,1,1.8463); (3.8494,1.1837,2.1449); (1.2567,1,1.6564); (2.5369,1,1.7662); (3.192 3,1.2,2.1361); (3.726,1.103,1.5158); (7.8752,3.9678,2.3173); (5.3621,3.4552,2.235); (3.5299,1.7097,1.6986); (1.1432,1,1.6466); (6.64 54,2.2434,2.3396); (18.001,20.205,2.5557); (2.873,1.2083,2.0249); (4.959,1.0839,1.4041); (16.298,7.877,2.5118); (1.1286,1,1.8169); ( 4.9557,3.6216,2.2186); (21.539,13.456,2.7049); (1.7081,1.0975,2.0714); (2.9013,1.0372,2.2278); (1.9439,1.0025,1.9659); (3.292,1.21 11,2.2832); (6.6937,2.2966,2.2558); (5.3549,3.0488,1.8951); (1.5426,1.0004,1.9491); (8.3117,4.0732,1.8672); (4.4836,2.5298,2.3327) ; (1.1595,1,1.8534); (4.3447,1.0134,2.0977); (6.0948,1.5253,1.9326); (1.4469,1,1.6415); (1.4293,1.0003,2.1753); (2.2416,1,1.6851); (4. 4425,1.0011,1.5379); (4.793,2.1145,2.2444); (1,1,1.9401); (1.6936,1.0111,1.9539); (14.75,5.8706,2.5869); (3.0295,1.4993,1.4513); (8. 3573,3.7099,2.1261); (3.9208,1.4212,2.2808); (1.1508,1,1.8286); (4.974,1.7095,1.9956); (4.9424,1.6355,2.1572); (1.6556,1.0215,2.14 55); (1.8071,1.0138,2.0157); (5.0815,1.2487,2.3244); (2.9977,1.9992,1.6573); (2.5331,1.1729,1.7437); (13.898,5.233,2.5067); (2.1555 , 1.364,1.7787); (4.1334,2.0008,1.9274); (17.279,6.6851,2.64); (2.7899,1.054,1.8481); (2.2781,1,1.8873); (1.2976,1,1.695); (4.8153,2.0 729,2.2787); (1.3394,1,1.7567); (8.0585,2.3986,2.2711); (1.4024,1,1.7119); (3.1149,1.3407,1.7821); (1.7143,1.1132,2.0068); (4.1443,1 . 1595,2.2524); (2.5041,1,1.4126); (2.4919,1.0151,1.5815); (3.9507,1.5629,1.816); (3.6496,1.0249,2.1841); (1.2503,1,1.7709); (1.8739, 1.0174,2.3327); (18.169,14.087,2.4673); (1.1137,1,1.831); (4.6781,1.9301,2.3234); (11.95,2.9194,2.4421); (2.9416,1.0672,1.7398); (5. 2563,1.4606,2.3396); (3.0141,1.1804,2.2075); (1.365,1,1.6714); (5.8731,3.8929,2.4881); (1.3618,1,1.2994); (1.1573,1,1.8217); (2.185 5,1.1674,1.7352); (6.7707,2.9377,2.1844); (1.5332,1,2.0697); (2.1409,1,1.2003);

(1.4444,1,1.5307); (9.6408,5.2268,2.1921); (3.3753,1 . 4355,2.1303); (2.5959,1.8175,1.8937); (1.0644,1,1.7306); (1.0936,1,2.0655); (3.2145,1.4379,2.2985); (5.3528,2.1476,1.6877); (3.649 6,1.0249,2.1841); (5.6549,1.4369,2.3535); (2.7088,1.1671,1.9827); (2.3768,1.1064,1.8994); (7.0067,1.2005,2.3557); (15.057,5.6717,2 . 4385); (5.0283,1.468,1.994); (9.5439,7.7388,2.6143); (15.978,9.3801,2.5303); (7.9588,4.8626,2.2095); (1.0464,1,2.1145); (23.829,16. 561,2.6671); (2.9068,1.7701,1.959); (5.6826,1.4138,2.1409); (1.1069,1,1.8632); (1.5319,1,2.0907); (1.8299,1,1.5323); (2.2606,1.0801, 1.9257); (1.0939,1,1.8322); (8.4539,2.9287,2.4081); (13.48,4.3218,2.6266); (2.7233,1.1746,1.9139); (5.9006,3.5902,2.1306); (1.6672, 1.0261,2.0933); (2.4234,1.0413,2.1554); (2.5712,1,1.4863); (1.7107,1.062,1.8561); (6.7146,2.603,2.3747); (2.0985,1.0421,1.9812); (6. 9735,3.1769,2.1089); (1.7041,1.0009,1.8162); (2.0194,1.0281,1.6602); (1.8834,1.0986,2.134); (41.918,37.451,2.8359); (6.2706,3.249 7,2.2205); (2.5623,1.098,2.3513); (2.8002,1.4993,1.3517); (1.472,1,2.1388); (9.707,6.455,2.0777); (4.911,2.9553,2.3826); (1.6583,1.02 04,1.7967); (29.616,23.256,2.729); (29.09,19.867,2.6953); (7.0331,2.59,2.2487); (2.4422,1.1883,1.9279); (4.3998,1.7292,2.241); (2.55 7,1.0918,2.0901); (1.6869,1.3108,2.0477); (2.3159,1.2663,1.873); (1.3885,1,1.5101); (2.4121,1.0353,1.7772); (10.147,4.07,2.4645); (3 . 6188,1.5958,2.2612); (4.6293,3.1642,1.9724); (1.7052,1.121,2.1609); (1.8457,1,1.3849); (9.3774,5.1738,2.3542); (2.6232,1.0476,1.9 443); (3.2521,1.479,2.3155); (4.7805,1.1892,2.2098); (3.5584,1.2202,2.121); (1.9199,1.0034,1.9188); (1.1089,1,1.8651); (6.5855,1.77 78,2.2853); (2.8448,1.1833,1.9443); (1.299,1,1.6428); (2.4149,1.1246,2.1385); (13.854,10.236,2.4697); (5.8041,1.5112,2.3273); (2.443 9,1.5082,1.9557); (2.4123,1.0432,2.1312); (6.8734,4.6187,2.4077); (4.2121,1.3963,2.1297); (5.0596,2.2369,2.1709); (1.5942,1.049,2. 0211); (5.8787,3.3044,2.2825); (7.9946,3.0349,2.4935); (1.9973,1.0028,2.0436); (1.169,1,1.7294); (1.7209,1,2.1554); (2.016,1.0249,1. 7787); (1.9141,1.0207,2.1659); (2.9778,1.0001,1.4112); (1.9987,1.1116,1.8968); (11.538,6.961,2.5457); (3.6504,1.4715,2.0181); (3.58 12,1,1.2285); (2.9393,1.1472,1.8171); (1.2109,1,1.822); (1.0645,1,1.9416); (1.9866,1.3226,1.8951); (4.2729,1.3863,2.1142); (4.918,3.3 479,2.0312); (1.5287,1,1.9804); (2.5487,1.0704,2.0178); (5.6691,2.1974,2.2491); (14.359,10.31,2.294); (4.6709,1.2854,1.4589); (10.5 12,3.1608,2.5316); (2.0944,1.9004,2.0921); (6.0918,3.1849,2.3487); (4.9644,3.93,2.2708); (1.5606,1.0033,1.8579); (4.4659,1.4547,2. 2033); (1.207,1,1.811); (1.2006,1,1.8835); (6.0884,4.3022,2.204); (1.1494,1,1.9346); (1.2006,1,1.8835); (1.2532,1,1.7199); (4.8958,1.0 9,2.4213); (3.2123,2.0977,2.1343); (6.3486,2.1717,2.1044); (1.1137,1,1.831); (4.7893,1.5442,2.2982); (9.4227,11.615,2.5143); (4.094, 2.0096,1.6767); (2.505,1.0187,1.7032); (4.0726,3.3956,1.9323); (3.4202,2.1095,2.2059); (2.696,1.0446,1.8279); (1.267,1,1.7808); (1.0 975,1,2.0542); (2.2352,1.043,1.6316); (4.8559,3.4452,1.9188); (1.195,1,1.7508); (6.2773,2.3856,2.1578); (2.2816,1.1009,1.9476); (1.2 255,1,1.803); (1.9254,1.0209,1.7414); (8.7928,4.1756,2.1575); (6.9226,3.2726,2.2457); (9.8995,5.3072,2.4182); (4.0189,1.2617,1.669 6); (3.6496,1.0249,2.1841); (1.4063,1,1.6759); (1.7695,1.0548,1.7732); (2.6922,1.2477,1.7457); (3.1261,2.0082,2.2101); (1.2172,1,1.7 298); (2.1479,1.3043,1.9896); (4.7863,1.2058,2.2182); (5.633,1.7767,2.3631); (1.8918,1,1.7459); (2.6392,1.107,2.0216); (1.0942,1,2.0 522); (4.4297,1.2512,2.2565); (4.3833,3.6815,2.2078); (1.9222,1.0097,2.0898); (7.9147,3.8315,2.4972); (3.9636,1.2749,1.9351); (3.58 , 1.0199,2.202); (1.6291,1,1.4222); (9.3069,6.0669,2.4608); (1.3206,1,1.549); (2.5398,1,1.6866); (1.0237,1,1.9093); (5.513,1.7048,2.16 96); (1.4494,1.0202,2.186); (2.214,1.4617,2.2043); (1.8583,1.0324,2.2307); (2.6043,1.4825,2.0708); (2.5443,1.0496,2.1975); (2.4356, 1.0567,2.0849); (6.1421,1.6865,2.4019); (7.443,2.8645,2.4081); (3.3289,1.1963,2.2127); (3.7299,1.2414,2.1591); (2.5093,1.7942,2.17 47); (40.298,37.241,2.8295); (3.1873,1.2203,1.9064); (2.0926,1.0178,1.9595); (3.0098,1.0844,1.9168); (3.747,1.7177,1.922); (1.6736, 1.0401,1.9408); (1.0484,1,1.9346); (7.3813,8.0153,2.3418); (2.7578,1.4983,2.1471); (9.0577,2.0012,2.4568); (1.3034,1,1.7989); (2.90 7,1.0923,2.2839); (7.3531,1.315,2.1725); (1.0854,1,2.0323); (3.5334,1.1792,1.8319); (2.325,1.1434,2.1665); (3.0969,1.3026,1.8871); ( 7.2238,2.1095,2.2777); (4.1946,1.5703,1.9967); (9.3597,2.296,2.1934); (6.1903,1.1639,2.1712); (1.1509,1,1.7618); (3.2929,1.0368,2. 0977); (3.4032,1.617,2.1569); (2.1083,1.0174,1.7528); (2.3244,1,1.3998); (17.208,15.893,2.6938); (2.6174,1.7523,1.8681); (1.3733,1, 2.1337); (1.6968,1.0074,1.8211); (1.6724,1.0506,1.8563); (2.1292,1.0248,2.1437); ( 1.6016,1.0574,1.9132); (2.5658,1.1937,2.1662); (1. 2945,1,1.6996); (2.4844,1.0131,1.6681); (2.2672,1.0082,1.5219); (5.5748,3.9406,1.8788); (1.4325,1,1.5578); (3.5056,3.0217,1.9848); (2.0043,1.1309,1.9628); (15.729,11.08,2.5714); (2.6658,1.9992,1.8887); (1.5047,1.0033,1.9641); (4.1788,1.9992,1.3703); (10.383,2.0 22,2.3194); (16.279,10.842,2.5824); (1.6427,1.0069,1.9428); (3.1412,1.0745,1.7612); (2.323,1.0117,1.8173); (3.9792,2.173,2.1264); (6 . 3185,2.9737,1.7781); (9.1926,6.9061,2.1541); (2.4123,1.0432,2.1312); (1.1323,1,1.9321); (5.8893,1.7449,2.2464); (6.9527,2.8766,2. 2108); (1.8834,1.0986,2.134); (5.5742,2.6059,2.2454); (3.3838,1.0124,1.8559); (3.3317,2.082,2.2072); (2.5455,1.2249,1.8117); (1.201 2,1,1.8277); (8.5878,4.6067,2.355); (1.6379,1.0055,1.8409); (6.4,7.1725,2.2241); (6.0454,2.8236,2.0595); (2.1808,1.1302,1.9284); (1. 4428,1,2.0728); (1.4675,1,1.4798); (1.7788,1.0089,1.8672); (3.1904,1.4789,2.2153); (7.9944,6.2446,2.3473); (2.3983,1.0598,2.2288); (1.2537,1,1.5056); (2.6879,1.0332,2.1816); (1.2751,1,1.6369); (2.048,1.0017,1.8342); (3.6797,1.2277,2.0372); (2.3209,1.0753,1.615); (5.4859,2.0641,2.4828); (4.2224,4.9682,2.105); (3.0996,1.0338,1.7005); (1.4714,1,2.2098); (1.3496,1,1.9235); (13.597,11.156,2.4718) ; (22.311,11.42,2.5936); (4.0097,2.2509,1.9125); (3.334,1.2314,2.2787); (2.5502,1.0734,2.0786); (2.2667,1.0527,1.5966); (1.2647,1,1. 7129); (5.242,2.3887,2.3613); (3.5977,1.0246,1.7322);

(2.3438,1.0003,1.4745); (3.2758,1.1763,1.7635); (2.8239,2.0063,2.0948); (1.65 49,1,1.2424); (2.8302,2.0058,1.9613); (1.029,1,1.9476); (10.019,1.9877,2.3269); (3.3131,2.0054,2.0378); (3.5812,1,1.2285); (1.6993,1 . 0109,2.0132); (7.6446,1.6096,2.3216); (1.1781,1,1.8123); (2.0668,1,2.0411); (4.4527,1.1356,1.7905); (1.5174,1.021,2.1006); (5.7225, 1.2629,1.6944); (1.4918,1,2.116); (2.6043,1.4825,2.0708); (1.909,1.1043,2.2491); (4.2605,1.2093,2.1889); (2.6047,1.0119,1.9499); (1. 1842,1,1.7664); (3.1867,2.0596,2.3305); (2.2781,1,1.8873); (8.3771,5.7693,2.4252); (2.2838,1.0311,1.5379); (4.9576,2.0558,2.3063); ( 2.6418,1.2869,2.1325); (3.1904,3.9945,1.6038); (4.3776,1.5726,2.1133); (2.3516,1.2231,2.0517); (3.3116,1.1289,1.9872); (2.3137,1.0 446,1.6825); (2.837,1.7667,2.0717); (8.7383,7.1337,2.1816); (4.2721,2.5215,2.098); (3.8968,1.434,2.2898); (1.9174,1.0196,1.7804); ( 9.7279,5.1882,2.4856); (6.2459,2.1042,2.3248); (1.1313,1,2.017); (4.1135,3.1737,2.2264); (2.5398,1.0305,1.4332); (1.8507,1.006,1.8 459); (44.544,47.523,2.8793); (1.5874,1.034,2.0763); (1.634,1,1.1913); (3.8006,2.0309,1.7465); (12.258,11.911,2.2898); (1.5635,1,1.5 588); (1.267,1,1.7808); (10.428,1.5721,2.3169); (2.1659,1.0016,1.6855); (2.4093,1.0479,2.1403); (13.911,11.491,2.2504); (1.1486,1,1. 806); (25.796,21.117,2.7245); (4.432,1.3576,2.0531); (1.8493,1.022,1.789); (4.5145,1.9595,2.1303); (1.158,1,1.6533); (2.6257,1.9992, 2.0528); (6.1318,1.6879,2.3539); (1.2012,1,1.8277); (6.8572,2.6133,2.3073); (2.5429,1.127,1.8961); (1.2479,1,1.7772); (3.9291,1.101 6,2.2667); (3.4424,2.0146,1.7382); (14.235,5.4966,2.6229); (1.1366,1,1.7392); (6.0809,2.1716,2.0295); (1.1869,1,1.6451); (7.2765,2.0 692,2.3334); (6.7744,1.1155,2.1715); (1.2255,1,1.803); (2.515,1,1.7961); (1.4877,1,1.5736); (3.5061,1.4363,2.1943); (3.1467,1.2538,2. 2241); (3.3322,1.9992,1.4732); (4.3651,2.3742,2.1743); (1.062,1,2.1246); (9.9071,3.1204,2.2619); (1.2479,1,1.7772); (5.0209,1.3808, 1.9463); (1.6432,1.0077,1.8534); (1.115,1,1.9506); (1.4686,1,1.5425); (5.4926,1.6924,1.7569); (2.7316,1.0111,2.1258); (11.429,8.0126 , 2.3309); (8.3239,2.6296,2.3371); (2.1094,1.7015,1.8391); (14.366,6.9949,2.4326); (2.0313,1.3303,2.17); (5.49,2.0346,2.4629); (8.954 3,2.8833,2.3066); (4.4328,1.6622,2.0436); (1.1122,1,1.9289); (4.2187,4.7277,2.212); (2.5866,1.0336,1.8744); (1.6497,1,1.8612); (6.20 48,2.1546,2.0455); (2.4838,1.0166,1.8217); (3.4472,1.3401,1.7705); (7.0158,3.7493,1.9173); (1.6354,1.0656,2.0522); (3.2376,1.2317, 2.0837); (9.0466,11.514,2.2278); (2.9347,1,1.7048); (8.1735,3.8233,2.2447); (11.781,6.3283,2.2585); (2.0062,1,1.9962); (25.754,15.33 4,2.5838); (2.9205,1.0757,2.1819); (3.0241,1.8849,1.9833); (4.4528,3.1609,2.1455); (2.9347,1,1.7048); (2.2687,1.2363,1.9698); (1.71 73,1,1.8117); (3.0755,1.3198,2.0249); (1.8273,1.0057,1.8545); (4.4849,2.3766,1.7528); (1.3266,1,1.7406); (3.1852,1.3174,1.76); (1.07 72,1,2.0455); (11.086,2.1 077,2.3769); (1.6981,1,1.7772); (2.1376,1.1109,1.6879); (2.7408,1.4266,1.7512); (1.0899,1,2.0983); (6.0672, 1.8825,1.8288); (1.1063,1,1.8262); (1.0918,1,2.0298); (3.8752,1.3456,1.533); (4.7713,2.4545,2.0547); (2.8479,1.002,1.7546); (5.3542, 3.9007,2.3403); (1.0332,1,1.9064); (7.9186,2.615,2.2321); (1.0948,1,1.8259); (1.198,1,1.753); (1.8071,1.0009,2.0464); (3.5444,1.7315 , 2.2056); (5.3455,3.4029,2.2014); (3.3832,2.3049,2.174); (2.9811,1.1624,2.2222); (2.6176,1.9411,1.8947); (1.7493,1,1.3128); (3.9507, 1.5629,1.816); (4.8408,1.9136,2.2582); (1.3455,1,1.6266); (5.7179,5.4978,2.1276); (2.7608,1.195,2.3087); (1.9074,1.1112,1.8704); (6. 7633,4.2089,2.2929); (1.8481,1.007,2.2078); (2.9327,1.0766,2.3073); (4.1817,2.0453,2.1409); (5.7006,2.2621,1.4722); (3.6496,1.024 9,2.1841); (3.6795,1.3631,2.1825); (16.068,10.23,2.5635); (2.102,1,1.2431); (2.5712,1,1.4863); (7.4025,4.3854,2.3687); (3.4125,1.250 9,2.0575); (1.1639,1,1.7948); (1.7344,1,1.7753); (5.6188,3.0905,2.1467); (3.5406,1.4508,2.1584); (1.4804,1,1.6027); (4.6055,1.3871,1 . 8586); ( 1.1313,1,2.017); (2.0256,1,1.3246); (1.1128,1,1.7534); (3.0827,1.7047,1.9509); (3.7682,1.2901,2.2307); (1.4926,1,1.6039); (2. 3006,1.0406,1.8623); (3.425,1.1941,2.1303); (5.9377,1.277,1.8534); (30.857,27.911,2.7529); (1.4325,1.0049,1.9451); (2.216,1.0191,2 . 0797); (1.0724,1,1.9262); (3.7246,1.0812,1.3646); (3.1115,2.1406,2.1734); (1.1166,1,1.7869); (3.4185,1.0491,1.9011); (2.5851,1.7782 , 2.2763); (4.704,3.0547,2.1258); (1.2346,1,1.6432); (12.465,4.4196,2.4762); (1.9473,1.0399,1.8386); (2.9405,1.6174,1.7563); (6.7638, 2.143,2.4058); (3.5409,1.2465,2.0063); (1.1636,1,1.7057); (1.5714,1,1.4282); (2.5547,1.0488,1.8438); (1.2356,1,1.7139); (3.5928,3.06 02,2.2434); (1.9093,1.0089,1.7901); (2.9143,1.1118,1.852); (1.6679,1.0252,1.97); (1.6651,1,1.4623); (5.838,1.7565,1.7954); (3.0031,1. 9992,1.7424); (6.0025,5.0524,2.1982); (4.4109,2.6704,2.1921); (5.6095,2.7956,2.3436); (3.5643,1.1608,1.8628); (3.3721,2.4988,2.18 1); (20.179,4.5197,2.5971); (5.8659,1.84,2.2964); (1.8901,1.0782,1.883); (10.178,4.1908,2.45); (1.2489,1,1.7544); (1.9598,1,1.3717); ( 2.6002,1.1285,2.2294); (8.5274,2.3133,2.2255); (5.5305,4.9552,2.2968); (2.2201,1.0301,1.6923); (4.979,2.0763,1.8949); (6.1032,5.84 57,2.1297); (1.3369,1,1.7451); (2.0642,1.0569,1.7294); (9.5201,7.8543,2.505); (3.9615,1.0225,2.2017); (2.4159,1.1345,1.9028); (3.7 522,1.3008,2.0276); (5.7343,2.1411,2.2518); (23.117,17.808,2.5514); (1.5246,1.003,1.9346); (2.6647,1.3601,1.8504); (2.779,1.2637,2 . 0956); (4.5299,2.1871,2.0369); (2.0531,1.0112,1.9112); (1.9185,1.0884,1.8468); (1.723,1.0016,1.8132); (3.6496,1.0249,2.1841); (1.49 31,1.0418,2.0195); (2.0669,1.004,1.8667); (1.6284,1,1.3974); (1.6021,1,1.9391); (4.1993,1.9339,2.1367); (2.0285,1.1884,1.8954); (1.8 765,1,1.3979); (3.509,1.7184,1.7993); (2.4598,1.0109,2.2687); (8.1014,2.7455,2.3223); (2.0333,1.0538,1.9631); (5.6486,1.6573,2.204 3); (2.4212,1.0011,2.0694); (2.9281,1.2743,2.0235); (2.2561,2.146,2.0052); (1.0661,1,1.8559); (1.9956,1,1.6978); (6.9971,3.0632,2.38 29); (1.8421,1.1833,1.9428); (4.0821,1.0885,2.1876); (1.0415,1,1.9654); (5.2685,1.2816,2.0114); (17.874,5.4079,2.6097); (1.6259,1.01 77,1.9476); (3.181,1.1508,2.2739); (6.1083,1.2844,2.3041); (5.9518,3.2734,2.288); (8.4629,3.5524,2.2114); (13.166,6.3207,2.4897); (6 . 7946,2.1253,2.3191); (2.9057,1,1.7424); (6.6135,5.5167,1.8637); (6.0366,4.5247,2.2189); (3.4349,1.2235,1.8847); (5.6557,1.6238,2. 2684); (4.2998,2.191,2.3385);

(4.5982,2.0536,1.8178); (2.3562,1.0361,2.187); (3.237,1.2049,2.1089); (13.499,4.7103,2.3309); (4.8684 , 1.5116,2.2954); (1.388,1,2.1077); (6.147,6.1203,1.8097); (1.3171,1,1.6398); (1.9505,1.0421,2.0855); (1.0386,1,2.091); (4.5599,1.2379 , 2.0489); (3.8831,1.5281,2.1978); (1.185,1,1.7833); (2.7812,1.2443,1.8556); (4.4849,2.3766,1.7528); (3.321,2.0821,2.2777); (1.1225,1 , 1.9649); (1.4447,1,1.6162); (16.315,5.1717,2.4236); (9.79,2.4595,2.2311); (12.174,9.3961,2.1609); (2.4281,1.0311,1.4052); (1.4918,1, 2.116); (1.1529,1,1.6362); (2.2367,1.055,2.1184); (9.3252,4.5128,1.8273); (3.485,3.6131,2.246); (3.4005,1.1837,2.1443); (1.47,1,1.641 5); (1.2608,1,1.7261); (10.919,5.3137,2.5295); (1.5255,1.0152,1.9969); (1.2945,1,1.6996); (4.7239,1.194,1.8871); (3.1354,2.3309,2.02 3); (3.2889,1.2053,2.1772); (2.8002,1.4993,1.3517); (3.1015,1.0485,1.6652); (4.1793,1.8862,2.0939); (1.4745,1,2.1337); (1.7892,1,1.3 438); (1.6681,1.014,1.9433); (5.3825,3.6477,2.2541); (4.5504,1.9992,1.4705); (3.9051,3.0416,2.0433); (2.6648,1.0421,1.9814); (1.908 , 1.1072,1.9421); (1.6757,1.0445,2.0629); (1.3903,1,1.6652); (4.9946,2.435,1.976); (5.6354,1.5243,2.2248); (8.6497,11.566,2.3338); (1. 6651,1,1.4623); (3.3701,1.2604,2.1628); (6.0777,3.6526,2.3928); (3.4235,1.7808,2.202); (8.451,2.7797,2.5223); (2.9261,1.0024,1.548 3); (2.6664,1,1.2798); (1.0936,1,2.0655); (3.229,1.2641,2.105); (1.71,1.0086,1.8545); (2.6296,3.9945,1.776); (3.315,1.7913,2.1606); (4. 9739,1.5645,1.4445); (2.4965,1.0045,1.4616); (1.0283,1,2.0875); (2.2474,1.2429,2.1306); (7.0479,4.5734,2.3698); (3.37,1.1512,2.102 7); (3.8077,1.2541,2.1461); (1.5511,1,1.9426); (1.4305,1,1.4674); (3.2979,1.2692,2.2248); (11.313,2.8997,2.4504); (4.1388,1.5814,2.2 297); (26.056,11.001,2.6804); (2.1412,1.1466,1.7402); (1.9621,1,1.4295); (4.476,2.3409,2.4318); (2.0421,1.2354,2.1934); (10.605,2.67 77,2.4742); (8.9128,4.4931,2.0652); (3.104,2.0009,1.678); (8.0163,1.3877,2.313); (3.1785,1.9992,1.7503); (2.9172,2.0046,1.9724); (1. 0975,1,2.0542); (5.0353,4.7006,1.8925); (1.8741,1.0541, 1.8362); (1.9775,1.0129,2.1319); (2.9328,1.0748,1.8913); (4.6892,1.4936,2.3 141); (2.0986,1.0067,1.9341 ); (2.7088,1.0493,1.7787); (2.198,1.1936,1.6729); (2.3905,1.082,2.1358); (3.7674,2.0051,2.076); (2.8665, 2.0079,2.1425); (4.1873,1.8145,2.0009); (1.1335,1,2.1322); (15.431,5.1429,2.5754); (7.4325,2.2084,2.5001); (6.0451,2.3778,1.9501); (8.181,2.8041,2.3694); (4.4474,1.6069,1.9641 ); (7.6606,4.955,2.4754); (2.9621,1.3937,1.7651); (10.45,8.4386,2.2971); (2.5042,1.754 6,2.3216); (3.1793,3.0999,1.9338); (22.918,17.166,2.6563); (2.6024,1.0478,1.7166); (2.4036,1.0201,1.7372); (5.1116,2.5704,2.4389); ( 4.5963,2.0356,1.4512); (1.7817,1,1.5107); (2.756,1.9992,1.8536); (1.2527,1,1.6373); (7.4728,4.6353,2.2354); (8.7902,5.0318,2.1157); (8.6423,5.1754,1.7182); (5.933,2.9202,2.2915); (1.2006,1,1.8835); (1.8422,1.048,1.7793); (2.7683,2.1262,2.203); (3.1466,1.0449,2.22 84); (3.3293,2.0862,1.7316); (3.9522,1.2191,2.1486); (1.7465,1.0012,1.773); (21.403,15.189,2.5785); (6.7187,2.6128,1.8973); (10.366 , 2.4938,2.3777); (3.5785,1.4767,2.2732); (2.3357,1.1181,2.0584); (4.7268,1.9992,1.3015); (45.803,46.655,2.9603); (6.7568,2.9258,1. 8812); (6.609,2.5072,2.4259); (8.6117,1.9145,2.3155); (1.5439,1.0012,1.9134); (2.6244,1.0463,2.1325); (2.9543,1.9992,1.7376); (6.57 38,3.5171,2.2238); (3.5846,1.8251,2.1195); (2.5694,1.0457,1.7952); (1.4481,1,2.0001); (9.2967,3.3369,2.2891); (1.7413,1.0096,1.848 8); (1.5258,1,1.7123); (3.5013,1.419,2.0378); (1.3641,1,2.1068); (2.0915,1.0223,1.9213); (4.3858,1.585,2.1911); (1.1684,1,1.7846); (3. 4424,1.8758,1.8518); (1.642,1.0006,1.7429); (5.0015,1.8533,2.0757); (1.4293,1.0003,2.1753); (3.7426,1.0736,2.307); (11.721,3.7859, 2.4461); (4.1179,1.0739,1.7972); (5.2685,2.4398,2.3363); (1.2934,1,1.6511); (1.185,1,1.8262); (1.5484,1.0025,1.9235); (2.8911,1.1471 , 2.3867); (1.5209,1.0146,2.0411); (4.3947,1.346,2.2301); (2.7089,1.3776,1.9768); (4.7719,2.0219,1.5528); (2.9199,1,1.4703); (7.3012, 4.4716,2.0768); (2.0323,1,1.6681); (1.0873,1,1.7536); (5.0038,3.3011,2.2307); (9.2197,5.0117,2.2811); (1.6062,1,1.599); (4.5201,2.99 9,2.0706); (4.076,2.1527,1.7514); (1.8012,1,1.6403); (14.123,8.4814,2.1972); (2.5686,1.1222,1.903); (3.3965,1.9992,1.5562); (6.9663, 3.5209,2.265); (4.4321,1.2441,1.9096); (1.1684,1,1.7846); (2.4056,1,1.212); (8.2224,4.4579,2.2982); (4.3515,1.1355,1.5601); (1.2626, 1,1.7697); (2.916,2.2599,2.0892); (1.5212,1,2.0167); (1.3991,1,1.529); (5.2099,1.246,2.3447); (1.5809,1,1.4534); (3.4014,1.07,2.0618) ; (1.2826,1,1.7818); (2.7912,1.9992,2.0663); (31.287,29.416,2.8804); (1.3765,1,1.6098); (1.1212,1,1.8284); (1.9775,1.0129,2.1319); (4. 1745,1.6286,2.2179); (4.6056,2.288,2.2884); (2.2667,1,1.5718); (5.1753,3.0147,2.2407); (2.0422,1.1129,1.7382); (1.1366,1,1.7392); (1 . 2541,1,1.7211); (2.8816,1.0681,2.1291); (29.003,35.443,2.8493); (1.6466,1.0128,1.7306); (3.6496,1.0249,2.1841); (2.562,1.0486,1.9 724); (6.0086,4.0637,2.4641); (14.7,5.2151,2.3837); (1.9793,1.0083,1.7223); (2.4617,1.1085,2.0991); (2.8012,1.0459,2.1743); (4.0471 , 1.1856,2.152); (1.2319,1,1.6542); (2.3418,1.0628,1.876); (4.672,1.6564,2.3502); (6.379,1.716,2.4042); (3.421,1.4621,2.214); (4.9404, 1.6688,2.3993); (1.5225,1.0063,2.0146); (2.2897,1.0009,1.7823); (2.2837,1.0606,2.1358); (2.1004,1.0208,2.1486); (1.0586,1,1.7816); (2.46,1.0033,2.2059); (1.2766,1,1.6226); (2.3841,1.0165,2.1056); (2.9898,2.531,2.1352); (3.5115,1.104,1.6043); (1.9566,1.0076,2.080 6); (2.3315,1.0467,2.1918); (3.7531,2.0401,1.9893); (2.563,1.0234,2.0991); (2.3493,1.0758,1.8025); (3.5299,1.7097,1.6986); (6.5014, 3.5724,2.0178); (15.382,6.9569,2.6335); (1.2742,1,1.5724); (16.709,7.9999,2.6642); (2.7444,1,1.6661); (11.816,4.2743,2.2992); (4.585 , 5.0225,2.2301); (13.585,5.5789,2.458); (16.465,7.8613,2.3003); (3.9742,1.9304,2.2694); (2.7591,1.0031,1.5855); (13.814,7.9858,2.4 385); (3.1525,1.9992,1.589); (1.1664,1,1.8384); (5.9544,1.3408,1.9267); (5.629,1.6711,2.355); (3.0533,1.2612,1.7463); (1.974,1.3549, 2.2407); (2.2039,1.0117,1.5469); (1.8255,1.1662,2.1612); (5.0858,2.2203,2.2514); (13.185,3.3483,1.9775); (1.687,1.0609,2.1163); (4.4 464,2.3913,2.2344); (1.2019,1,1.7396); (1.8291,1.1825,1.9675); (5.6626,2.5579,2.313); (1.3871,1,1.7219); (2.2939,1.5213,2.0881); (1. 1867,1,1.8053); (15.022,6.0286,2.4429); (3.5046,2.0108,1.7991);

(10.349,6.2855,2.452); (1.1626,1,1.7594); (2.6677,1,1); (1.3266,1,1. 7406); (1.1247,1,1.836); (4.6308,1.3386,2.2968); (1.115,1,1.9506); (5.0755,1.4613,1.5006); (1.5494,1.0024,1.9721); (1.7157,1.0318,1. 8377); (2.9284,1.6094,1.8138); (2.5492,1.0355,1.9796); (3.2297,1.0001,1.4774); (6.2614,2.5551,2.2189); (2.4627,1.0347,2.2176); (2.0 182,1.0026,1.8591); (13.025,3.6169,2.4584); (2.0697,1.2663,1.8866); (4.4103,3.6875,1.7542);

(4.5627,1.3171,2.0157); (1.365,1,1.671 4); (1.8832,1.0114,2.1112); (1.108,1,1.8628); (10.306,9.9904,2.3327); (3.2803,1.5398,2.3425); (19.231,15.037,2.3066); (1.3975,1,1.65 04); (5.7236,4.7679,2.3447); (5.2099,1.246,2.3447); (3.2758,1.1763,1.7635); (2.0739,1.0196,2.0878); (3.243,1.0001,1.3464); (1.1183,1 , 1.6637); (6.2262,1.5188.2.3784); (7.6596,4.8033,2.3739); (1.9262,1.0191,1.7829); (32.462,21.171,2.6704); (1.2346.1,1.6432); (14.53 9,3.8909,2.4588); (5.3195,1.523,2.0055); (5.1065,1.2884,2.3572); (7.9066,5.0651,2.1816); (3.7127,1.1214,2.268); (3.0295,1.4993,1.4 513); (1.6377,1,1.8579); (1.8803,1,1.392); (3.554,2.0019,1.5139); (5.6257,2.1726,2.0124); (2.4056,1,1.212); (4.5024,2.9919,2.2347); (5 . 0079,1.5246,2.3484); (2.645,1.6283,2.1778); (1.6296,1.0121,1.961); (5.4872,1.0917,1.8956); (4.7031,2.6926,2.1835); (2.5973,1.2037 , 1.4178); (2.6648,1.0421,1.9814); (2.9186,1.0008,1.8112); (9.9378,6.2505,2.4464); (7.0717,3.8945,2.3349); (3.385,1.2072,2.2595); (1. 0712,1,2.1151); (2.3642,1.0449,2.0615); (5.6083,2.6334,2.1325); (39.493,31.203,2.8194); (1.3166,1,1.69); (46.299,36.165,2.7809); (4. 6401,1.2976,2.1693); (3.3428,2.0221,2.1959); (1.7758,1.01117,1.9062); (2.3539,1.0003,1.6641); (1.2484,1,1.8438); (28.483,21.546,2.6 751); (3.8851,1.0501,1.8328); (1.3549,1,1.6668); (1.8106,1.036,1.8676); (3.0231,1.0132,2.1969); (3.2608,1.0003,1.2277); (1.8187,1,1. 5265); (1.4802,1.0125,2.1331); (1.3979,1.0013,2.0657); (7.8671,3.0571,2.287); (2.329,1.1906,1.827); (3.5314,2.2157,1.7972); (2.3079 , 1.2401,1.8447); (1.3484,1,1.5452); (2.7147,1.2696,2.3059); (3.6006,1.088,1.9983); (1.4636,1,1.6027); (4.2678,2.5118,1.958); (2.3819, 1.0019,2.1529); (2.0487,1,1.7835); (1.2214,1,1.7188); (2.6623,1.1154,2.1756); (5.7962,1.6445,2.0464); (1.8029,1.0003,2.1994); (1.317 1,1,1.6398); (8.6269,1.4522,2.3531); (1.5688,1.0068,1.9181); (8.4075,1.6577,2.3517); (6.0587,3.6758,2.0436); (13.081,9.2426,2.5596 ); (3.6496,1.0249,2.1841); (4.6101,2.2946,2.3735); (4.4054,1.8208,2.3223); (2.0353,1.6042,1.8095); (1.1036,1,1.9436); (3.0371,1.055 1,2.2575); (3.0067,1.0856,1.9983); (2.1959,1.117,2.14); (3.0063,1.2834,2.288); (1.2006,1,1.8835); (3.5091,1.226,1.9856); (3.8147,1.50 37,2.2404); (2.7444,1,1.6661); (11.173,5.5676,2.521); (2.8353,1.1343,1.9086); (1.0111,1,1.958); (1.8599,1.0011,2.2069); (7.181,3.387 3,2.3119); (8.139,2.5175,2.2387); (11.532,6.273,2.1489); (1.8071,1.0009,2.0464); (1.9594,1.0222,1.9608); (3.5982,1.5567,1.9466); (6. 1403,2.9982,1.8513); (6.0125,2.5546,2.3631); (8.0061,2.7333,2.2811); (2.2575,1.1264,2.0222); (4.7648,1.8516,1.8413); (6.5794,2.51 29,2.2954); (1.3238,1,1.5916); (3.0591,1.2468,1.74); (8.4393,4.2172,2.0956); (2.0353,1.6042,1.8095); (1.1326,1,1.8461); (24.479,13.5 53,2.6647); (1.3898,1,1.6224); (3.3405,2.02,1.8568); (4.1948,5.6048,2.0298); (2.4445,1.2899,2.148); (1.5258,1,1.7123); (2.48,2.1267, 1.9237); (3.0141,1.2501,1.816); (2.7176,1.0154,1.7487); (2.3529,1.0927,1.7606); (1.2302,1,1.8041); (1.4374,1.0185,2.0309); (14.627, 4.4815,2.4244); (11.488,5.5326,2.6088); (4.3238,2.1148,2.064); (1.6816,1.0306,1.9641); (5.2869,1.6521,1.4901); (3.3554,1.1951,1.75 03); (3.6202,2.0078,2.1765); (2.713,1.2021,1.7802); (6.3146,3.0908,2.216); (1.6796,1.0318,2.1328); (6.2551,2.8553,2.422); (3.8393,2. 1827,2.0007); (1.1526,1,1.6921); (1.2917,1,1.6915); (2.3068,1.0671,2.0863); (3.5291,1.2608,1.9603); (3.3002,2.319,2.1972); (3.1266, 1.2625,2.1376); (8.6806,3.7515,2.1991); (6.7673,2.806,2.4042); (2.2946,1.0361,1.4983); (2.278,1.0333,1.6058); (4.0957,1.6927,1.811 ); (1.7235,1.0525,2.0375); (1.1966,1,1.7884); (3.1703,1.0661,2.2049); (2.1535,1.0124,2.0691); (1.4588,1,1.412); (1.3099,1,1.6314); (1. 2489,1,1.7544); (1.3878,1,1.735); (7.7746,3.6113,2.3546); (1.1321,1,1.9499); (2.0735,1.0842,2.0159); (4.0042,1.5293,1.5789); (1.5226 , 1,1.957); (7.6564,3.53,2.0595); (3.5741,1.7709,2.1734); (2.2087,1,1.7119); (7.3399,3.4652,2.4166); (2.103,1.0086,1.7336); (8.0386,2. 634,2.5151); (3.7536,1.9992,1.5721); (2.7591,1.2753,2.05); (14.579,9.8703,2.6138); (1.0167,1, 1.9565); (6.5493,4.1 002,2.1603); (2.43 34,1.3311,1.557); (1.9413,1.0172,1.9888); (16.044,410.729,2.5618); (3.2475,4.5342,2.235); (3.2543,2.0052,1.7901); (6.0475,2.342,1.4 765); (10.568,4.7579,2.3792); (1.1457,1,1.7457); (15.881,11.272,2.5505); (2.1857,1.0815,1.9428); (3.1535,1.0428,2.2404); (1.8287,1,1 . 9481); (8.9362,5.4706,2.4035); (4.7114,1.6156,2.3126); (1.3099,1,1.6314); (9.8126,2.8813,2.4984); (8.5353,3.9028,2.3765); (1.2945,1 , 1.6996); (3.3784,1.0092,1.8064); (6.2723,2.5051,2.3262); (2.9177,1.3043,2.2407); (1.0322,1,1.9962); (2.4883,1.0657,1.8493); (1.079, 1,1.7069); (4.1242,1.1746,2.3066); (3.1917,1.2192,1.8935); (58.463,88.935,2.9122); (5.7608,3.2743,1.7914); (4.7495,1.1039,1.5398); (1.6508,1.0586,2.0954); (1.7518,1,2.1784); (1.3538,1,1.8651); (1.374,1,1.6819); (4.2773,2.3523,2.148); (3.7821,1.1338,2.0052); (1.10 1,1,1.6975); (1.3034,1,1.7989); (4.3521,1.4005,2.2437); (4.0155,1.0638,2.2756); (2.368,1.135,1.8017); (2.6225,1.0191,1.784); (1.0918 , 1,2.0298); (2.1037,1.0019,1.5992); (4.3551,1.7299,1.9496); (2.5133,1.2571,2.0154); (4.1436,3.0594,1.9532); (1.9413,1,1.7288); (3.04 05,2.9976,2.0924); (2.8432,1.3447,2.0309); (7.0903,4.2616,1.8306); (1.4818,1,1.4894); (1.3033,1,1.755); (4.7464,1.6671,2.2825); (6.3 76,1.6303,2.2585); (1.0899,1,2.0983); (1.5226,1,1.957); (1.2906,1,1.6933); (1.8017,1,1.6319); (3.4659,1.4257,2.165); (5.2547,2.7244, 2.1437); (8.3371,6.17,2.2873); (3.6851,1.5661,2.1765); (6.1031,2.6431,2.2599); (1.8517,1.0069,1.9967); (6.4718,1.0936,2.1391); (2.7 885,1.0982,1.7716); (19.858,7.4654,2.594); (1.2321,1,1.7188); (1.9566,1.0076,2.0806); (2.3099,1.0681,1.7818); (2.3903,1.0544,2.051 7); (1.1511,1,1.8797); (8.8973,2.6562,2.2964); (4.6163,1.151,1.534); (2.2511,1.2662,1.9552); (4.4431,1.2709,2.0369); (1.8073,1.0016,1.5133); (2.9284,1.0457,2.2846); (6.1928,3.5268,2.2735); (2.3053,1.1446,1.8612); (2.3591,1.0486,2.0001); (1.1069,1,1.836); (1.8571, 1,1.5035);

(5.8287,2.7983,1.7082); (2.5588,1.1597,1.7952); (9.3139,6.3325,2.4275); (8.6995,7.1534,2.4877);
(3.1256,2.004,1.7404); ( 1.1247,1,1.836); (3.6309,1.3061,2.3158); (2.0484,1.0034,1.7635); (7.2108,5.9999,2.5206);
(5.6211,2.9979,1.687); (5.2267,2.2311,2.3 92); (2.2559,1.0637,1.6582); (1.5974,1.0093,1.965 1); (1.3455,1,1.6266);
(4.2769,1.1673,2.3724); (5.0103,1.7704,1.9309); (1.9577,1,1. 7487); (3.0464,3.9945,1.7418); (3.1894,2.7029,1.9122);
(4.6727,2.2373,2.194); (2.713,1.2021,1.7802); (5.19,2.2204,2.2866); (3.2122, 1.5911,2.0916); (8.9043,2.5346,1.9962);
(2.551,1.0357,1.777); (3.2408,1.1689,2.0257); (1.2934,1,1.6511); (1.198,1,1.753); (4.4624,1.9 992,1.4009);
(1.6209,1.0776,2.1584); (3.2616,1.3987,2.2248); (5.3209,1.9657,2.3498); (15.381,7.1406,2.2255);
(3.2385,2.2197,2.132 5); (3.5314,1.0191,2.202); (2.6961,1.1775,2.2324); (1.3484,1,1.5452); (4.1377,1.7455,2.1628);
(1.1664,1,1.8384); (2.4759,1.0342,1.94 53); (3.0716,1.3475,2.2394); (1.1486,1,1.806); (2.3457,1.1041,1.956);
(2.1758,1,1.6959); (3.5275,1.0456,1.8711); (5.6884,2.4599,2.27 63); (4.7457,2.1567,2.2377); (5.4312,5.2033,2.1391);

Part II: IP values of Sinomenine-related genes

| Gene | IP value | NodeRank | Betweenness | Closeness |
|---|---|---|---|---|
| 581 | 6.1834 | 6.5855 | 1.7778 | 2.2853 |
| 596 | 18.265 | 15.978 | 9.3801 | 2.5303 |
| 6347 | 6.3687 | 6.2869 | 2.4204 | 1.9626 |
| 6348 | 5.3322 | 4.7031 | 2.6926 | 2.1835 |
| 6352 | 10.809 | 7.3813 | 8.0153 | 2.3418 |
| 958 | 5.7661 | 5.7343 | 2.1411 | 2.2518 |
| 941 | 2.1418 | 1.9577 | 1 | 1.7487 |
| 942 | 2.1418 | 1.9577 | 1 | 1.7487 |
| 1027 | 12.133 | 11.173 | 5.5676 | 2.521 |
| 1278 | 11.793 | 11.816 | 4.2743 | 2.2992 |
| 9170 | 1.6419 | 1.3043 | 1 | 1.6992 |
| 1906 | 4.2158 | 4.1058 | 1.6685 | 1.9448 |
| 2258 | 4.0385 | 4.2605 | 1.2093 | 2.1889 |
| 2335 | 33.203 | 24.564 | 22.378 | 2.541 |
| 3383 | 3.9573 | 3.883 | 1.5318 | 2.2127 |
| 8519 | 1.9824 | 1.7413 | 1.0096 | 1.8488 |
| 3596 | 1.91 | 1.6399 | 1.0176 | 1.7724 |
| 3553 | 5.0708 | 4.6755 | 2.3195 | 2.2548 |
| 3569 | 2.0616 | 1.8385 | 1.0171 | 2.0677 |
| 3576 | 7.1515 | 5.8846 | 4.1141 | 2.2384 |
| 3714 | 2.2781 | 2.1347 | 1.0015 | 1.7633 |
| 4322 | 4.8742 | 4.5299 | 2.1871 | 2.0369 |
| 4792 | 13.31 | 11.538 | 6.961 | 2.5457 |
| 5228 | 5.7783 | 6.1318 | 1.6879 | 2.3539 |
| 5743 | 14.065 | 8.6497 | 11.566 | 2.3338 |
| 7040 | 17.191 | 13.854 | 10.236 | 2.4697 |
| 7124 | 7.5393 | 7.443 | 2.8645 | 2.4081 |
| 7133 | 4.281 | 4.1738 | 1.689 | 2.2444 |
| 7157 | 102 | 58.463 | 88.935 | 2.9122 |
| 7412 | 8.2158 | 7.461 | 3.8938 | 2.1731 |
| 7422 | 10.992 | 9.8995 | 5.3072 | 2.4182 |

(note: all genes are indicated by Entrez GeneID of NCBI)

Part III: IP values of Matrine-related genes

| Gene | IP value | NodeRank | Betweenness | Closeness |
|---|---|---|---|---|
| 7356 | 1.4644 | 1.0724 | 1 | 1.9262 |
| 7157 | 102 | 58.463 | 88.935 | 2.9122 |

(continued)

| Gene | IP value | NodeRank | Betweenness | Closeness |
|------|----------|----------|-------------|-----------|
| 581 | 6.1834 | 6.5855 | 1.7778 | 2.2853 |
| 355 | 9.0518 | 7.6606 | 4.955 | 2.4754 |
| 4893 | 16.583 | 15.486 | 7.3526 | 2.6097 |
| 604 | 5.4139 | 5.8041 | 1.5112 | 2.3273 |
| 3265 | 24.805 | 21.456 | 13.027 | 2.6747 |
| 1026 | 11.776 | 10.919 | 5.3137 | 2.5295 |
| 1032 | 2.9654 | 3.0231 | 1.0132 | 2.1969 |
| 596 | 18.265 | 15.978 | 9.3801 | 2.5303 |
| 567 | 8.4655 | 7.3012 | 4.4716 | 2.0768 |
| 595 | 16.668 | 17.066 | 5.6075 | 2.5557 |
| 4609 | 17.158 | 17.874 | 5.4079 | 2.6097 |
| 5111 | 17.66 | 16.465 | 7.8613 | 2.3003 |
| 3725 | 35.049 | 29.09 | 19.867 | 2.6953 |
| 891 | 7.8855 | 8.0061 | 2.7333 | 2.2811 |
| 7015 | 5.2026 | 4.7123 | 2.4805 | 2.34 |
| 1019 | 14.143 | 13.166 | 6.3207 | 2.4897 |
| 5743 | 14.065 | 8.6497 | 11.566 | 2.3338 |
| 6774 | 39.944 | 29.049 | 27.518 | 2.8158 |
| 6777 | 13.096 | 13.48 | 4.3218 | 2.6266 |
| 4089 | 37.634 | 29.616 | 23.256 | 2.729 |
| 4314 | 6.339 | 6.2773 | 2.3856 | 2.1578 |
| 836 | 40.873 | 28.304 | 29.846 | 2.7015 |
| 1027 | 12.133 | 11.173 | 5.5676 | 2.521 |
| 947 | 1.6313 | 1.2906 | 1 | 1.6933 |
| 3674 | 12.773 | 12.104 | 5.4543 | 2.4275 |
| 5925 | 35.666 | 28.483 | 21.546 | 2.6751 |
| 1029 | 6.8854 | 7.2238 | 2.1095 | 2.2777 |
| 2526 | 8.6439 | 6.147 | 6.1203 | 1.8097 |
| 3684 | 8.7155 | 8.1735 | 3.8233 | 2.2447 |
| 4830 | 12.863 | 11.532 | 6.273 | 2.1489 |
| 7076 | 2.6601 | 2.5605 | 1.0888 | 1.9433 |
| 7408 | 9.4162 | 8.7928 | 4.1756 | 2.1575 |
| 56259 | 1.4583 | 1.0644 | 1 | 1.7306 |
| 174 | 1.4808 | 1.0939 | 1 | 1.8322 |
| 945 | 1.9842 | 1.7518 | 1 | 2.1784 |
| 7040 | 17.191 | 13.854 | 10.236 | 2.4697 |
| 1277 | 13.361 | 13.499 | 4.7103 | 2.3309 |
| 59 | 5.7242 | 4.3833 | 3.6815 | 2.2078 |
| 960 | 17.682 | 14.131 | 10.669 | 2.4803 |
| 7422 | 10.992 | 9.8995 | 5.3072 | 2.4182 |
| 2258 | 4.0385 | 4.2605 | 1.2093 | 2.1889 |
| 7124 | 7.5393 | 7.443 | 2.8645 | 2.4081 |
| 3569 | 2.0616 | 1.8385 | 1.0171 | 2.0677 |
| 1956 | 54.812 | 40.298 | 37.241 | 2.8295 |
| 1281 | 7.5153 | 8.3156 | 1.7904 | 2.2447 |
| 1285 | 5.6574 | 5.8105 | 1.8817 | 2.1285 |
| 4313 | 17.966 | 16.406 | 8.4075 | 2.3031 |
| 2335 | 33.203 | 24.564 | 22.378 | 2.541 |
| 4035 | 26.782 | 18.001 | 20.205 | 2.5557 |
| 3383 | 3.9573 | 3.883 | 1.5318 | 2.2127 |

Part IV: IP values of Honokiol-related genes

| Gene | IP value | NodeRank | Betweenness | Closeness |
|---|---|---|---|---|
| 7157 | 102 | 58.463 | 88.935 | 2.9122 |
| 7124 | 7.5393 | 7.443 | 2.8645 | 2.4081 |
| 3586 | 1.5605 | 1.198 | 1 | 1.753 |
| 598 | 13.987 | 13.585 | 5.5789 | 2.458 |
| 836 | 40.873 | 28.304 | 29.846 | 2.7015 |
| 6647 | 5.6279 | 5.0143 | 2.7821 | 2.1921 |
| 5443 | 3.0752 | 2.5093 | 1.7942 | 2.1747 |
| 581 | 6.1834 | 6.5855 | 1.7778 | 2.2853 |
| 596 | 18.265 | 15.978 | 9.3801 | 2.5303 |
| 796 | 3.1253 | 3.243 | 1.0001 | 1.3464 |
| 6750 | 3.2638 | 3.2758 | 1.1763 | 1.7635 |
| 4842 | 7.5971 | 6.9663 | 3.5209 | 2.265 |
| 3553 | 5.0708 | 4.6755 | 2.3195 | 2.2548 |
| 4353 | 3.1482 | 3.0533 | 1.2612 | 1.7463 |
| 5743 | 14.065 | 8.6497 | 11.566 | 2.3338 |
| 3791 | 15.49 | 15.469 | 5.6757 | 2.5692 |
| 4318 | 16.366 | 15.381 | 7.1406 | 2.2255 |
| 4843 | 6.6256 | 5.8787 | 3.3044 | 2.2825 |

Part V: IP values of Luteolin-related genes

| Gene | IP value | NodeRank | Betweenness | Closeness |
|---|---|---|---|---|
| 207 | 41.581 | 30.857 | 27.911 | 2.7529 |
| 367 | 45.012 | 29.003 | 35.443 | 2.8493 |
| 581 | 6.1834 | 6.5855 | 1.7778 | 2.2853 |
| 596 | 18.265 | 15.978 | 9.3801 | 2.5303 |
| 598 | 13.987 | 13.585 | 5.5789 | 2.458 |
| 836 | 40.873 | 28.304 | 29.846 | 2.7015 |
| 841 | 17.184 | 15.391 | 8.3992 | 2.4836 |
| 891 | 7.8855 | 8.0061 | 2.7333 | 2.2811 |
| 595 | 16.668 | 17.066 | 5.6075 | 2.5557 |
| 958 | 5.7661 | 5.7343 | 2.1411 | 2.2518 |
| 1020 | 11.262 | 8.6995 | 7.1534 | 2.4877 |
| 1026 | 11.776 | 10.919 | 5.3137 | 2.5295 |
| 1576 | 4.8152 | 4.8053 | 1.7683 | 1.8434 |
| 1906 | 4.2158 | 4.1058 | 1.6685 | 1.9448 |
| 1956 | 54.812 | 40.298 | 37.241 | 2.8295 |
| 355 | 9.0518 | 7.6606 | 4.955 | 2.4754 |
| 2353 | 16.587 | 16.151 | 6.5691 | 2.5419 |
| 2308 | 7.0002 | 5.8731 | 3.8929 | 2.4881 |
| 2309 | 4.8637 | 5.1355 | 1.4512 | 2.4042 |
| 3480 | 28.588 | 22.918 | 17.166 | 2.6563 |
| 3596 | 1.91 | 1.6399 | 1.0176 | 1.7724 |
| 3565 | 1.552 | 1.1869 | 1 | 1.6451 |
| 3569 | 2.0616 | 1.8385 | 1.0171 | 2.0677 |
| 3576 | 7.1515 | 5.8846 | 4.1141 | 2.2384 |

(continued)

| Gene | IP value | NodeRank | Betweenness | Closeness |
|---|---|---|---|---|
| 3725 | 35.049 | 29.09 | 19.867 | 2.6953 |
| 5594 | 65.085 | 45.803 | 46.655 | 2.9603 |
| 1432 | 28.895 | 23.829 | 16.561 | 2.6671 |
| 5599 | 25.144 | 21.539 | 13.456 | 2.7049 |
| 4792 | 13.31 | 11.538 | 6.961 | 2.5457 |
| 4843 | 6.6256 | 5.8787 | 3.3044 | 2.2825 |
| 142 | 8.5803 | 7.8752 | 3.9678 | 2.3173 |
| 5335 | 26.35 | 24.989 | 11.23 | 2.5846 |
| 9536 | 7.8641 | 6.069 | 5.0018 | 1.8073 |
| 5743 | 14.065 | 8.6497 | 11.566 | 2.3338 |
| 6774 | 39.944 | 29.049 | 27.518 | 2.8158 |
| 7124 | 7.5393 | 7.443 | 2.8645 | 2.4081 |
| 7128 | 2.9995 | 3.0067 | 1.0856 | 1.9983 |
| 7157 | 102 | 58.463 | 88.935 | 2.9122 |
| 7422 | 10.992 | 9.8995 | 5.3072 | 2.4182 |

Part VI: matrix of shortest paths among Sinomenine-Matrine -related genes

4,4,4,4,3,4,5,5,3,2,4,4,4,1,4,4,4,4,4,3,4,3,4,4,4,3,3,4,3,3,3 1,1,3,3,3,2,4,4,2,3,4,3,3,2,3,2,4,3,3,2,3,3,1,2,
1,2,2,3,0,3,2 0,1,4,4,3,3,4,4,2,3,5,4,3,3,3,3,4,3,4,3,4,4,2,3,2,3,3,4,1,3,3 2,2,3,3,3,3,3,3,2,3,4,4,2,2,2,3,3,3,3,3,4,4,
2,2,2,3,3,3,1,3,2 2,1,4,2,2,3,4,4,2,3,3,3,2,2,2,3,3,2,2,2,3,2,2,3,2,2,3,2,2,2 3,2,4,2,2,3,4,4,3,4,5,3,3,3,3,4,5,2,2,2,4,4,
2,3,3,3,2,3,2,4,3 2,1,4,2,2,2,3,3,2,3,3,3,2,3,2,3,3,2,2,2,4,3,2,2,2,2,2,2,2,2 2,2,3,3,3,3,4,4,2,3,4,3,3,3,3,3,4,3,3,3,4,4,
2,2,2,3,2,3,1,3,2 3,3,4,3,3,3,4,4,2,3,5,3,4,2,4,4,5,3,3,3,4,3,3,3,2,3,3,2,4,3 1,0,4,3,3,3,4,4,2,3,4,3,3,3,3,3,4,4,3,3,2,3,
4,2,3,2,3,2,3,1,3,3 4,3,4,4,4,4,4,4,3,3,5,2,4,3,4,4,5,2,4,4,4,3,3,3,4,3,4,4,3,4,3 3,2,3,2,2,3,3,3,1,4,4,3,3,3,3,4,4,2,2,2,
3,4,2,2,3,3,2,2,2,3,2 3,2,3,3,3,3,3,3,2,3,4,3,3,3,3,4,4,3,3,3,3,3,3,2,3,2,2,2,2,3,2 3,2,4,3,3,2,4,4,2,3,5,3,4,3,4,4,4,4,3,3,
3,4,4,2,3,3,3,3,3,2,3,3 3,2,3,1,1,3,4,4,2,3,4,3,3,2,3,4,4,1,1,1,3,4,2,2,3,2,1,2,2,3,2 2,3,4,3,3,3,4,4,1,3,4,4,3,3,3,4,4,3,
3,3,4,3,3,3,3,3,2,3,3,2,4,3 2,3,4,4,3,2,4,4,2,3,4,4,3,3,3,4,4,3,3,4,4,3,2,3,3,3,2,3,2,3,3 3,2,4,3,3,3,4,4,1,3,4,2,3,3,3,4,4,
3,3,3,3,4,3,3,3,2,2,3,2,3,3 2,2,4,4,4,2,4,4,3,4,3,3,3,3,3,4,4,4,3,4,3,2,3,0,3,2,2,1,3,3 3,2,2,2,2,3,3,3,2,3,3,3,2,2,2,3,3,
2,2,2,4,3,2,1,3,3,2,2,2,3,1 1,2,2,3,3,3,3,2,2,2,3,4,3,2,3,2,3,3,3,3,3,4,3,2,2,3,3,2,2,2,3,2 3,2,3,2,2,3,4,4,1,2,4,3,3,1,3,4,
4,2,2,2,2,3,2,3,3,1,2,3,2,3,3 3,3,1,3,3,3,5,5,3,2,4,4,3,2,3,4,4,3,4,3,4,2,2,3,3,2,2,3,2,3,3 2,1,4,3,3,2,3,3,2,2;4,3,2,2,
3,4,4,2,3,3,3,3,3,2,3,2,2,2,2,3,2 2,2,4,3,2,3,3,3,0,3,4,3,3,2,3,4,4,3,3,3,3,4,2,3,3,2,3,3,2,3,3 4,4,4,4,3,5,5,5,3,4,5,
4,4,4,4,5,5,4,5,4,6,5,4,4,5,4,4,5,4,4,4 3,3,3,3,2,3,4,4,2,1,4,4,3,1,3,4,4,3,4,3,4,2,3,3,3,2,3,3,2,3,2 3,2,4,2,2,2,2,4,4,2,3,4,
3,3,2,3,4,3,2,2,2,4,4,2,3,3,3,2,3,2,2,3 3,3,4,3,3,3,4,4,2,3,4,3,3,2,4,4,5,3,3,3,3,4,3,2,3,2,3,3,2,4,2 4,4,3,4,3,4,5,5,4,4,
5,5,4,4,2,3,5,5,5,5,6,4,4,4,4,4,4,3,4,4 3,3,3,3,3,3,4,4,3,3,4,3,3,3,1,3,4,4,4,4,5,3,3,3,3,3,3,2,3,3 3,3,3,4,3,3,5,5,
3,2,4,4,3,2,4,4,3,4,4,4,5,3,3,3,3,3,4,3,2,2,3 4,4,2,3,4,4,5,5,4,2,4,3,3,2,4,3,4,2,4,4,3,3,3,4,4,2,3,4,3,3,4 2,3,3,3,3,3,4,4,
3,2,4,4,3,2,3,3,3,3,4,4,5,3,3,3,3,2,4,3,3,2,3 4,4,3,4,4,4,5,5,4,3,4,4,4,4,3,4,4,4,4,5,4,6,4,4,4,4,3,4,4,4,3,4 4,4,2,2,2,4,4,4,
4,4,5,5,4,4,4,5,4,4,4,3,6,3,3,3,4,3,4,3,3,4,3 3,3,4,4,3,3,3,3,3,3,4,4,3,3,3,4,3,3,3,4,5,3,2,3,3,3,3,3,3,3 3,3,3,1,3,3,5,5,
2,2,4,3,3,2,3,4,4,2,3,3,3,2,3,2,3,0,2,3,2,3,2 3,3,3,3,2,3,4,4,3,2,3,4,3,1,3,3,3,2,4,2,4,3,3,3,3,2,2,3,2,2,2 3,3,4,3,3,4,4,4,
3,2,5,3,3,2,4,3,5,3,3,3,3,4,3,3,3,3,3,2,4,3 3,3,2,2,2,3,3,3,3,1,2,4,2,1,2,4,4,3,4,2,4,2,2,2,3,2,3,3,2,3,2 3,3,3,3,2,3,4,
4,3,2,3,4,2,2,2,4,4,3,3,3,4,3,3,1,3,2,3,2,2,3,0 3,3,4,4,3,3,4,4,3,3,3,4,0,3,2,4,4,3,4,3,5,4,3,2,3,3,3,3,3,2 3,2,3,2,2,3,
4,4,3,2,4,4,3,2,3,4,4,2,2,2,3,3,2,3,2,2,0,1,2,3,3 4,3,4,2,2,4,4,4,3,4,5,4,4,3,4,5,4,2,0,2,4,4,3,4,3,2,3,3,4,3 2,2,3,3,2,
2,3,3,2,2,3,3,1,2,1,3,3,2,3,3,4,3,2,1,2,2,2,2,2,1 3,3,3,3,2,3,4,4,3,2,3,3,3,2,3,3,3,4,2,3,3,3,3,3,2,3,3,2,2,2 4,3,3,3,3,
3,5,5,3,2,4,4,3,1,3,4,4,3,4,2,4,3,4,4,3,2,2,3,3,3,3 3,3,3,2,3,3,5,5,3,2,4,4,2,2,3,4,3,1,3,3,4,2,3,3,3,1,2,3,2,2,3 3,3,3,3,
2,3,4,4,2,1,3,3,3,0,3,3,3,3,3,2,3,2,3,3,3,2,2,3,2,2 3,3,3,3,3,2,4,4,3,2,4,3,3,2,3,3,3,2,3,3,4,1,2,3,2,2,2,2,2,2,2 3,3,4,4,
3,3,4,4,3,3,3,4,2,3,0,4,4,3,4,4,5,4,3,2,3,3,3,3,3,2

Part VII: matrix of shortest paths among Sinomenine- Honokiol-related genes

1,1,3,3,3,2,4,4,2,3,4,3,3,2,3,2,4,3,3,2,3,3,1,2,1,2,2,3,0,3,2 3,2,3,2,2,3,4,4,3,2,4,4,3,2,3,4,4,2,2,2,3,3,2,3,2,2,
0,1,2,3,3 4,4,4,4,4,4,6,6,4,3,5,4,4,3,4,4,5,2,4,4,4,3,4,4,4,3,4,4,4,4,3 1,1,3,3,3,3,3,3,2,3,4,4,3,3,3,3,4,2,3,3,3,4,2,3,2,3,
2,3,1,3,3 2,1,4,3,3,2,3,3,2,2,4,3,2,2,3,4,4,2,3,3,3,3,3,2,3,2,2,2,2,3,2 2,1,4,4,4,3,4,4,3,4,5,4,4,3,3,5,4,4,3,4,4,2,3,2,3,
3,4,1,4,3 4,3,3,3,3,4,4,4,3,3,4,4,3,2,3,4,4,3,3,3,5,4,3,2,4,2,3,3,3,3,2 0,1,4,4,3,3,4,4,2,3,5,4,3,3,3,3,4,3,4,3,4,4,2,3,2,
3,3,4,1,3,3 1,0,4,3,3,3,4,4,2,3,4,3,3,3,3,3,4,3,3,2,3,4,2,3,2,3,2,3,1,3,3 6,6,7,6,5,6,6,6,5,6,6,3,5,5,5,5,6,7,5,6,6,7,6,5,5,5,5,6,6,5,6,5 3,3,5,5,4,4,4,4,4,5,5,5,4,4,4,4,4,5,4,4,5,5,3,4,3,4,4,4,2,5,4 3,2,4,3,3,3,3,3,3,4,4,4,3,3,3,4,3,3,3,3,4,4,2,
3,3,3,3,3,2,3,3 3,3,4,2,2,3,5,5,3,3,5,4,3,3,3,4,4,0,2,2,4,3,3,3,4,2,2,3,3,3,3 4,4,4,4,4,4,6,6,4,4,6,4,4,4,4,5,6,4,4,4,5,4,4,
4,4,4,4,4,5,4 2,2,4,4,4,2,4,4,3,3,4,3,3,3,3,3,4,4,4,3,4,3,2,3,0,3,2,2,1,3,3 3,3,3,3,3,2,2,3,3,2,2,4,3,3,2,2,3,3,3,3,3,4,2,2,
1,2,3,3,2,2,2,1 3,3,3,3,3,4,4,4,3,1,3,4,3,1,3,4,3,3,3,1,4,2,3,3,3,2,3,4,2,2,3 3,2,4,3,3,2,4,4,3,3,4,3,3,3,3,3,4,4,3,3,3,4,3,
2,3,2,3,3,2,3,3,3

Part VIII: matrix of shortest paths among Sinomenine- Luteolin-related genes

2,2,3,3,3,2,4,4,1,2,4,3,3,2,3,4,3,3,3,3,4,3,2,2,3,3,2,2,2,2,2 2,2,3,2,2,2,4,4,2,3,4,3,2,2,2,4,4,2,2,2,3,3,2,2,2,2,
2,2,2,3,2 0,1,4,4,3,3,4,4,2,3,5,4,3,3,3,3,4,3,4,3,4,4,2,3,2,3,3,4,1,3,3 1,0,4,3,3,3,4,4,2,3,4,3,3,3,3,4,3,3,2,3,4,2,3,
2,3,2,3,1,3,3 1,1,3,3,3,3,3,3,2,3,4,4,3,3,3,3,4,2,3,3,3,4,2,3,2,3,2,3,1,3,3 2,1,4,3,3,2,3,3,2,2,4,3,2,2,3,4,4,2,3,3,3,3,3,
2,3,2,2,2,2,3,2 2,1,4,3,3,2,3,3,1,2,4,4,3,3,3,4,4,3,3,3,3,2,3,3,2,2,2,2,3,3 2,3,4,3,3,3,4,4,1,3,4,4,3,3,3,4,4,3,3,3,4,3,3,
3,3,2,3,3,2,4,3 3,2,3,2,2,3,3,3,1,4,4,3,3,3,4,4,2,2,2,3,4,2,2,3,3,2,2,2,3,2 3,3,4,4,4,0,4,4,3,3,4,3,3,3,3,3,4,4,3,4,4,3,3,
3,2,3,3,2,2,3,3 2,2,4,3,3,3,3,1,2,4,4,3,3,3,4,4,3,3,3,3,2,3,2,2,3,3,1,3,3 2,2,3,3,3,3,4,4,2,3,4,3,3,3,3,3,4,3,3,3,4,4,
2,2,2,3,2,3,1,3,2 4,3,5,4,4,4,5,5,4,4,4,4,4,3,3,5,5,4,4,4,5,5,4,4,2,4,3,4,3,4,4 4,3,5,4,4,3,4,4,3,3,4,0,4,3,4,4,5,4,4,4,4,
4,3,4,3,3,4,3,3,4,4 2,2,3,3,2,2,3,3,2,2,3,3,1,2,1,3,3,2,3,3,4,3,2,1,2,2,2,2,2,2,1 2,2,3,3,3,3,3,3,2,3,4,4,2,2,2,3,3,3,3,3,4,
4,2,2,2,3,3,3,1,3,2 3,2,3,1,1,3,4,4,2,3,4,4,3,3,3,4,1,1,1,3,4,2,2,3,2,1,2,2,3,2 3,3,3,3,3,3,3,4,4,2,3,4,4,3,2,3,4,4,3,3,3,3,
3,2,3,3,2,3,3,2,3,3 3,3,4,3,3,3,4,4,2,3,4,3,3,2,3,4,4,3,3,3,3,4,2,3,3,2,2,3,2,3 3,2,3,3,2,3,3,3,2,2,2,3,1,2,2,3,3,3,3,3,
4,3,2,1,3,3,3,3,2,2,1 4,4,4,4,4,4,4,4,4,3,5,5,4,3,4,4,0,4,4,4,6,4,3,4,4,4,4,4,4,1,4 4,4,4,4,4,4,4,4,4,5,5,5,4,4,4,5,2,5,4,
5,6,5,3,4,4,5,4,4,4,3,4 4,3,4,2,2,4,4,4,3,4,5,4,4,3,4,5,4,2,0,2,4,4,3,3,4,3,2,3,3,4,3 3,2,2,2,2,4,5,5,3,2,4,4,3,2,4,4,4,2,2,
0,4,3,3,3,3,2,3,2,3,3 3,2,3,1,1,3,4,4,2,3,4,3,3,2,3,4,4,1,1,1,3,4,2,2,3,2,1,2,2,3,2 2,1,3,2,2,2,3,3,2,3,4,3,2,2,2,3,4,2,
2,2,3,3,2,2,2,1,2,1,3,2 2,1,3,2,2,3,4,4,2,3,4,4,2,3,3,3,3,2,2,1,3,4,2,2,2,2,2,2,1,2,2 2,1,3,2,2,3,4,4,2,3,3,3,3,3,2,3,4,2,
2,2,3,3,2,2,2,2,1,2,1,3,2 2,2,3,3,3,3,3,3,2,3,4,3,3,3,3,3,3,3,3,3,4,3,0,3,2,3,2,3,1,3,3 3,2,4,3,3,2,4,4,3,3,4,3,3,3,3,4,4,
3,3,3,4,3,2,3,2,3,3,2,3,3,3 2,1,4,4,3,2,4,4,3,3,5,4,3,3,3,3,2,4,3,4,3,2,3,2,3,3,4,1,2,3 3,2,3,3,2,3,3,3,3,2,3,3,3,3,2,3,1,
3,3,3,3,3,5,3,2,2,2,3,3,3,2,2,2 3,3,5,5,5,3,5,5,4,4,5,4,4,4,4,4,5,5,5,4,5,4,3,4,1,4,3,3,2,4,4 2,2,4,4,4,2,4,4,3,3,4,3,3,
3,3,3,4,4,4,3,4,3,4,2,3,0,3,2,2,1,3,3 3,2,2,2,2,3,3,3,2,3,3,3,2,2,2,3,3,2,2,2,4,3,2,1,3,3,2,2,2,3,1 3,2,3,2,2,3,4,4,3,2,4,4,3,
2,4,4,3,2,3,4,4,2,2,2,3,3,2,3,2,2,0,1,2,3,3 4,3,4,4,4,2,4,4,3,4,5,4,4,4,4,5,4,3,4,4,5,4,2,4,3,3,2,2,3,4,4 1,1,3,3,3,2,4,4,2,3,
4,3,3,2,3,2,4,3,3,2,3,3,1,2,1,2,2,3,0,3,2 3,3,3,3,3,2,3,4,4,3,2,3,4,2,2,2,4,4,3,3,3,4,4,3,3,1,3,2,3,2,2,3,0

Embodiment 2: determination of effects on anti blood vessel endothelial cell migration of six medicines, based on Angiogenesis network

Experiment: applying the above-described method of the invention for determination of medicine action based on gene network to the screening of matrine and five other anti-Angiogenesis medicines (as main ingredients of QLY), and effects of the six medicines were basically determined with respect to the bioprocess of endothelial cell migration by evaluating correlation between a medicine (medicine-related gene subset) and a bioprocess (bioprocess-related gene subset), as indicated by equation (1)

$$ST = \frac{1}{2} \times \left( \frac{\sum_i VC_D(i) \times \exp\left(-\min(d_{i,j})\right)}{\sum_i VC_D(i)} + \frac{\sum_j VC_G(j) \times \exp\left(-\min(d_{j,i})\right)}{\sum_j VC_G(j)} \right) \qquad \text{Equ. (1)}$$

where ST (Score of Topology) was score of correlation between the subset of genes/gene products of a medicine action and the subset of genes/gene products of a bioprocess (such as endothelial cell migration), VC (Vertex Centrality) was the importance of the genes/gene products in the subset of genes/gene products of the medicine action or bioprocess in the network, where $VC_D(i)$ was the node importance score of node $i$ corresponding to genes/gene products in the subset of genes/gene products of a medicine action, and $VC_G(j)$ was the node importance score of node $j$ corresponding to genes/gene products in the subset of genes/gene products of a bioprocess. $d_{i,j}$ and $d_{j,i}$ were the shortest path from node $i$ to node $j$ and from node $j$ to node $i$ respectively.

A basic point of the method according to the present embodiment is to determine the topological correlation (including, such as, the length of the shortest path d between network nodes, and etc.) of the following two group of genes/gene products on a disease-related gene network (such as the above-mentioned Angiogenesis network):

-    a group of genes and/or gene products relating to a medicine action to be determined (a subset of medicine-related genes/gene products), and

- a group of genes and/or gene products relating to a bioprocess in a disease (a subset of bioprocess-related genes/ gene products).

According to an embodiment of the present invention, this determination included measuring the importance (indicated by VC) of a related node (gene/gene product) in the network (such as whether it was a key node, whether it was a node of strong connectivity, and etc.);

And the ST score expressed in Equ. (1) was obtained.

This score was used for ranking of the extent of correlation between a medicine action and a bioprocess, and for determining/predicting the effect of the medicine/component involved by comparison with ST scores of other medicines/components.

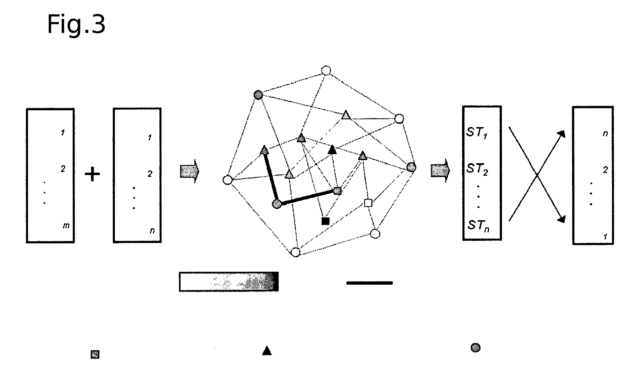

Fig. 3 is a schematic flowchart of an embodiment of the method. As a specific example, given a bioprocess participated by m genes/gene products, and n candidate medicine or components ($Drug_1$, ..., $Drug_n$).

Subset of genes/gene products corresponding to endothelial cell migration can be obtained from biological experiment or database like GO (http://www.geneontology.org/); subset of genes/gene relating to a medicine action can be obtained/improved by literature mining/collection in such as PubMed, CNKI, etc., or by Docking, biological experiments and/or etc.

Since the NIDA method of the invention has good robustness, difference in the subsets of genes/gene products used had relatively small effect on the outcomes of application of the NIDA method or was even negligible, as explained above.

Then, genes/gene products in the subset of genes/gene products corresponding to a bioprocess (such as endothelial cell migration) in Angiogenesis and genes/gene products in the subset of genes/gene products of each medicine were mapped onto the Angiogenesis gene network (which is published at and can be downloaded from: http://bioinfo.au.tsinghua.edu.cn/member/nzhang/download/angiogenesis.txt).

According to the definitions of NodeRank, Betweenness and Closeness, as explained above, their values for each of the nodes were calculated; and, to ensure comparability of data, these values were normalized by dividing respective minimum value. Finally, a 1893*3 matrix A was obtained, as given in "Part I: Noderank-Betweenness-Closeness values of Angiogenesis Network (Matrix A)", wherein the three values in the i-th parentheses were the noderank, betweenness and closeness values of the i-th gene listed in the webpage of:

http://bioinfo.au.tsinghua.edu.cn/member/nzhang/download/angiogenesis.txt

PCA (principal components analysis) on the three 1893*1 vectors formed by the three columns of the matrix A respectively was carried out in MATLAB. The commands in MATLAB were [COEFF, SCORE]=princomp(A); $VC$ = -A*COEFF(:,1), where the 1893*3 matrix formed by the three vectors was labeled A and the final node importance factor vector was labeled VC. (Of course, we can use only any two of the three vectors for PCA to obtain the IP value of node; if only Betweenness and Closeness are chosen, the corresponding program in MATLAB is: [COEFF, SCORE] = princomp (C); $VC$ = -A*COEFF(:,1), where C stands for the corresponding 1893*2 matrix).

Thus, the ST score of each medicine relating to the corresponding disease/treatment process was obtained on the basis of the node importance and length of the shortest path of the network corresponding to the disease/treatment, and then the corresponding ranking was obtained.

As an alternative approach, instead of mapping genes/gene products in subsets of bioprocess and medicine/ component to the gene network of corresponding disease (such as Angiogenesis network), they can also be mapped to any known global gene network (such as human global gene network, or an animal's global gene network in the case of veterinary drug research) to obtain substantially the same results of determination. Obviously, all such variations are within the scope of the present invention.

According to an embodiment of the present invention, EC migration as a bioprocess (GO number: 0043542) with close correlation to angiogenesis was chosen, and extent of actions by Matrine, Quercetin, Emodin, Evodiamine, Genistein, and Aconitine were determined. The screening by the NIDA method of the present invention showed (see Table 4) that matrine had strong correlation to the bioprocess of endothelial cell migration and the highest ranking among the six medicines chosen; matrine had the strongest ability in inhibiting endothelial cell migration, stronger than the other five medicine. This showed that matrine had remarkable effect on endothelial cell migration.

Experiments carried out by the inventors (as will be described below) supported the above results. And the ranking of inhibition ratios of the six medicines determined by the experiments were basically the same as that determined by NIDA method, showing that NIDA truly reflected the action of these medicines.

Table 4: extent of action by Matrine, Quercetin, Emodin, Evodiamine, Genistein, and Aconitine on the bioprocess of endothelial cell migration, as determined by NIDA method

| medicine | bioprocess | NIDA RANKING | Ranking by experiment results |
|---|---|---|---|
| Matrine | endothelial cell migration (GO number:0043542; limitation:Homo sapiens) | 1 | 1 |
| Quercetin | | 2 | 2 |
| Emodin | | 3 | 3 |
| Evodiamine | | 4 | 4 |
| Aconitine | | 5 | 6 |
| Genistein | | 6 | 5 |

Experimental determination of in vitro effect of Matrine and etc. on endothelial cell migration

Experiment: cell scratch experiments were used to quantitively measure the inhibition of medicines on migration. The principle of the experiment was to culture endothelial cells on a plate or in a culture dish; after cell fusion, a line was drawn with cell scraper in the central area so that cells within this line were mechanically removed, then the cells were further cultured and cell migration to the scrapped area without any cell was observed for determining in vitro effect of medicine on endothelial cell migration. Sterile operation had to be ensured during cell scrapping process. Human Umbilical Vein Endothelial Cells (HUVECs) available from Cascade Biologics (Portland, USA) were repeatably cultured with the supplier's criterions; Matrine, quercetin, emodin, evodiamine, genistein, and aconitine were purchased from National Institute for the Control of Pharmaceutical and Biological Products, and their experimental density were set at 10mM as indicated by literature. HUVECs were cultured on 12-plate coated by 0.1% gelatin, the cells were inactivated after 2 hours under the conditions of 5% $CO_2$, 37 °C and 10ug/ml of Mitonycin C. Cell scraper about 1mm in width was used to scrap on the fused surface of single-layer of cells. Standard endothelial cell growth medium (ECGM) free of medicine application was taken as control group. Pictures were taken at 6h-10h of medicine intervention by Nikon digital camera, with migration distance being scaled by configure software. The experiment was repeated five times. The anti-proliferation ability of the medicines was measured by Migration Inhibition Rate, determined as:

Migration Inhibition Rate = (migration distance of control group - migration distance of experimental group)/ migration distance of control group

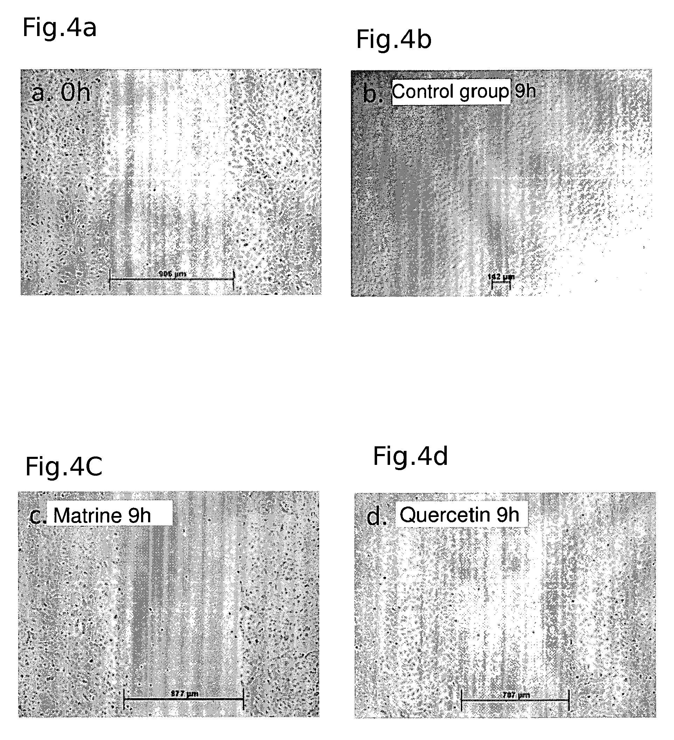

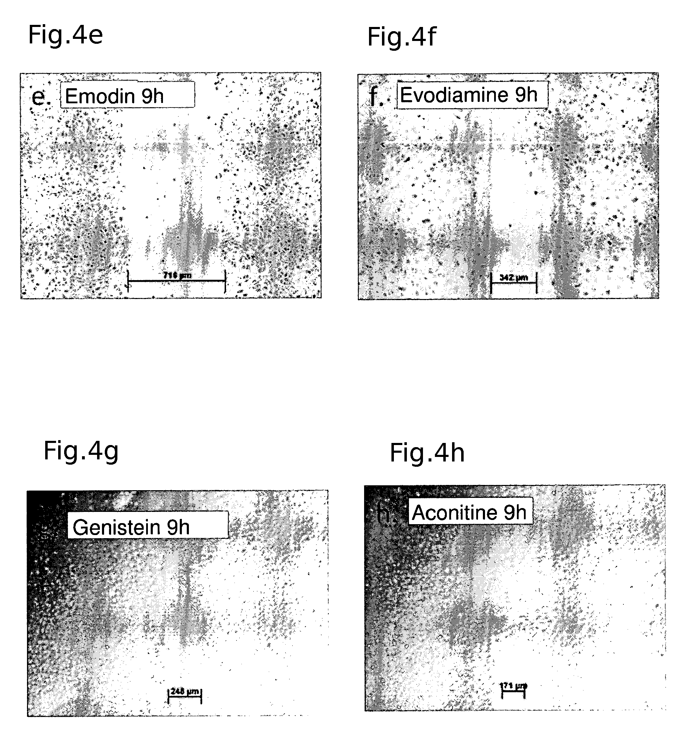

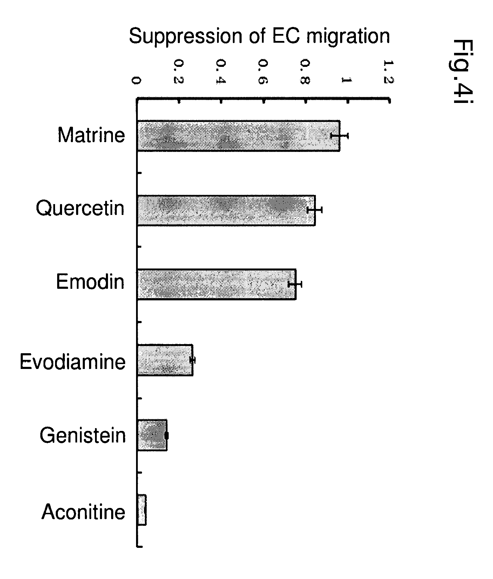

Results of the experiment: as shown in Fig. 4, Fig. 4a showed the scratch of 0h; Fig. 4b showed the result of the control group after 9h without medicine intervention, in which the scratch was seen to have reduced obviously; Fig. 4c to Fig. 4h showed the results of 9h intervention by matrine, quercetin, emodin, evodiamine, genistein, and aconitine respectively; Fig. 4i showed in histogram variance the inhibition of endothelial cell migration by each medicine. It was seen from the drawing that all six candidate anti-angiogenesi medicines had inhibited endothelial cell migration to different extent, with matrine having the strongest inhibition on endothelial cell migration.

The experimental results proved the correctness of the results of determination by NIDA method of the invention, and the extent of inhibition of the six medicines as obtained by the experiments were basically the same as the ranking obtained by NIDA method, showing that NIDA method of the invention truly determined the feature of actions of these medicines.

Related data, parameters, and intermediate data and etc. are listed in "Part IX" to "Part XXI"

Part IX: Matrine-related genes and corresponding $VC_D$ values

| Gene | $VC_D$ value | NodeRank | Betweenness | Closeness |
|---|---|---|---|---|
| 7356 | 6.6017 | 5.4903 | 3.7287 | 2.0068 |
| 7157 | 6.516 | 5.7608 | 3.2743 | 1.7914 |
| 581 | 2.9385 | 2.8448 | 1.1833 | 1.9443 |
| 355 | 3.1638 | 2.9621 | 1.3937 | 1.7651 |

(continued)

| Gene | $VC_D$ value | NodeRank | Betweenness | Closeness |
|---|---|---|---|---|
| 4893 | 1.4445 | 1.0464 | 1 | 2.1145 |
| 604 | 2.841 | 2.4439 | 1.5082 | 1.9557 |
| 3265 | 5.5235 | 5.7118 | 1.7911 | 2.2873 |
| 1026 | 1.8209 | 1.5255 | 1.0152 | 1.9969 |
| 1032 | 3.139 | 3.2608 | 1.0003 | 1.2277 |
| 596 | 9.2204 | 7.9588 | 4.8626 | 2.2095 |
| 567 | 2.1989 | 2.0323 | 1 | 1.6681 |
| 595 | 2.1215 | 1.8709 | 1.0717 | 1.8746 |
| 4609 | 1.8993 | 1.6259 | 1.0177 | 1.9476 |
| 5111 | 4.2837 | 3.9742 | 1.9304 | 2.2694 |
| 3725 | 7.0488 | 7.0331 | 2.59 | 2.2487 |
| 891 | 2.4525 | 2.2575 | 1.1264 | 2.0222 |
| 7015 | 1.6128 | 1.2664 | 1 | 1.7096 |
| 1019 | 6.5671 | 6.7946 | 2.1253 | 2.3191 |
| 5743 | 1.9179 | 1.6651 | 1 | 1.4623 |
| 6774 | 4.1736 | 3.5526 | 2.2603 | 2.1934 |
| 6777 | 2.84 | 2.7233 | 1.1746 | 1.9139 |
| 4089 | 35.049 | 29.09 | 19.867 | 2.6953 |
| 4314 | 2.4546 | 2.2816 | 1.1009 | 1.9476 |
| 836 | 1.5869 | 1.2325 | 1 | 1.7449 |
| 1027 | 2.8998 | 2.8353 | 1.1343 | 1.9086 |
| 947 | 2.0224 | 1.8017 | 1 | 1.6319 |
| 3674 | 4.6729 | 4.5125 | 1.8952 | 2.2926 |
| 5925 | 3.6487 | 3.8851 | 1.0501 | 1.8328 |
| 1029 | 4.2205 | 4.1946 | 1.5703 | 1.9967 |
| 2526 | 1.6516 | 1.3171 | 1 | 1.6398 |
| 3684 | 13.089 | 11.781 | 6.3283 | 2.2585 |
| 4830 | 2.0271 | 1.8071 | 1.0009 | 2.0464 |
| 7076 | 1.6652 | 1.3349 | 1 | 1.7245 |
| 7408 | 7.4038 | 6.9226 | 3.2726 | 2.2457 |
| 56259 | 1.4806 | 1.0936 | 1 | 2.0655 |
| 174 | 8.3541 | 8.4539 | 2.9287 | 2.4081 |
| 945 | 1.6797 | 1.3538 | 1 | 1.8651 |
| 7040 | 5.4139 | 5.8041 | 1.5112 | 2.3273 |
| 1277 | 4.6982 | 4.8684 | 1.5116 | 2.2954 |
| 59 | 2.1208 | 1.9222 | 1.0097 | 2.0898 |
| 960 | 5.0708 | 4.6755 | 2.3195 | 2.2548 |
| 7422 | 3.8873 | 4.0189 | 1.2617 | 1.6696 |
| 2258 | 2.6445 | 2.6047 | 1.0119 | 1.9499 |
| 7124 | 3.3173 | 3.3289 | 1.1963 | 2.2127 |
| 3569 | 3.1151 | 3.2297 | 1.0001 | 1.4774 |
| 1956 | 3.2244 | 3.1873 | 1.2203 | 1.9064 |
| 1281 | 2.6362 | 2.4772 | 1.1507 | 1.9406 |
| 1285 | 3.4975 | 3.3624 | 1.4363 | 1.8436 |
| 4313 | 2.2781 | 2.1347 | 1.0015 | 1.7633 |
| 2335 | 1.5817 | 1.2257 | 1 | 1.7447 |
| 4035 | 2.9762 | 2.873 | 1.2083 | 2.0249 |
| 3383 | 4.8623 | 4.5834 | 2.105 | 2.0869 |

Part X: Quercetin-related genes and corresponding $VC_D$ values

| Gene | $VC_D$ value | NodeRank | Betweenness | Closeness |
|------|------|------|------|------|
| 207 | 1.7431 | 1.4325 | 1.0049 | 1.9451 |
| 572 | 2.7095 | 2.1094 | 1.7015 | 1.8391 |
| 581 | 2.9385 | 2.8448 | 1.1833 | 1.9443 |
| 598 | 17.66 | 16.465 | 7.8613 | 2.3003 |
| 836 | 1.5869 | 1.2325 | 1 | 1.7449 |
| 841 | 2.4022 | 2.2803 | 1.0211 | 1.6005 |
| 6347 | 2.3159 | 2.1177 | 1.0803 | 2.1609 |
| 948 | 8.2138 | 6.9995 | 4.4391 | 2.4193 |
| 1026 | 1.8209 | 1.5255 | 1.0152 | 1.9969 |
| 1027 | 2.8998 | 2.8353 | 1.1343 | 1.9086 |
| 1277 | 4.6982 | 4.8684 | 1.5116 | 2.2954 |
| 1543 | 1.4298 | 1.0272 | 1 | 1.9391 |
| 1545 | 1.7387 | 1.4309 | 1 | 1.6823 |
| 1958 | 1.531 | 1.1595 | 1 | 1.8534 |
| 2064 | 2.7437 | 2.7444 | 1 | 1.6661 |
| 2065 | 4.9377 | 4.1876 | 2.6926 | 2.0971 |
| 2353 | 2.4229 | 2.2352 | 1.1069 | 2.0575 |
| 3091 | 2.3686 | 2.1857 | 1.0815 | 1.9428 |
| 3383 | 4.8623 | 4.5834 | 2.105 | 2.0869 |
| 3553 | 10.401 | 8.0775 | 6.5547 | 2.0519 |
| 3569 | 3.1151 | 3.2297 | 1.0001 | 1.4774 |
| 3576 | 6.5223 | 6.1965 | 2.7663 | 2.3947 |
| 3725 | 7.0488 | 7.0331 | 2.59 | 2.2487 |
| 5594 | 7.0537 | 6.7568 | 2.9258 | 1.8812 |
| 1432 | 3.3638 | 2.9068 | 1.7701 | 1.959 |
| 5599 | 2.0136 | 1.7081 | 1.0975 | 2.0714 |
| 4843 | 8.071 | 7.9946 | 3.0349 | 2.4935 |
| 4846 | 11.262 | 8.6995 | 7.1534 | 2.4877 |
| 5743 | 1.9179 | 1.6651 | 1 | 1.4623 |
| 5894 | 2.2002 | 2.0243 | 1.0116 | 1.9988 |
| 6401 | 1.4903 | 1.1063 | 1 | 1.8262 |
| 6721 | 2.4871 | 2.3398 | 1.0823 | 1.6453 |
| 6772 | 3.1451 | 3.0199 | 1.296 | 1.5533 |
| 7124 | 3.3173 | 3.3289 | 1.1963 | 2.2127 |
| 7157 | 6.516 | 5.7608 | 3.2743 | 1.7914 |
| 7412 | 1.4657 | 1.0741 | 1 | 1.7231 |
| 7422 | 3.8873 | 4.0189 | 1.2617 | 1.6696 |

Part XI: Emodin-related genes and corresponding $VC_D$ values

| Gene | $VC_D$ value | NodeRank | Betweenness | Closeness |
|------|------|------|------|------|
| 567 | 2.1989 | 2.0323 | 1 | 1.6681 |
| 596 | 9.2204 | 7.9588 | 4.8626 | 2.2095 |
| 637 | 4.7255 | 4.1271 | 2.435 | 2.0737 |
| 683 | 1.9574 | 1.7159 | 1.0009 | 1.873 |
| 836 | 1.5869 | 1.2325 | 1 | 1.7449 |

(continued)

| Gene | $VC_D$ value | NodeRank | Betweenness | Closeness |
|---|---|---|---|---|
| 6347 | 2.3159 | 2.1177 | 1.0803 | 2.1609 |
| 890 | 1.76 | 1.4588 | 1 | 1.412 |
| 891 | 2.4525 | 2.2575 | 1.1264 | 2.0222 |
| 1017 | 2.4108 | 2.0313 | 1.3303 | 2.17 |
| 1385 | 2.3387 | 1.8236 | 1.4653 | 1.9964 |
| 1436 | 17.625 | 14.359 | 10.31 | 2.294 |
| 1500 | 2.5426 | 2.368 | 1.135 | 1.8017 |
| 2335 | 1.5817 | 1.2257 | 1 | 1.7447 |
| 2353 | 2.4229 | 2.2352 | 1.1069 | 2.0575 |
| 3065 | 2.9272 | 2.9205 | 1.0757 | 2.1819 |
| 3308 | 2.9596 | 2.7089 | 1.3776 | 1.9768 |
| 3586 | 2.0271 | 1.8071 | 1.0009 | 2.0464 |
| 3667 | 1.5135 | 1.1366 | 1 | 1.7392 |
| 3717 | 1.7073 | 1.3898 | 1 | 1.6224 |
| 3725 | 7.0488 | 7.0331 | 2.59 | 2.2487 |
| 3845 | 3.2833 | 3.0295 | 1.4993 | 1.4513 |
| 3932 | 1.9564 | 1.683 | 1.0385 | 1.9846 |
| 5606 | 5.7242 | 4.3833 | 3.6815 | 2.2078 |
| 5608 | 3.8596 | 3.3428 | 2.0221 | 2.1959 |
| 5594 | 7.0537 | 6.7568 | 2.9258 | 1.8812 |
| 1432 | 3.3638 | 2.9068 | 1.7701 | 1.959 |
| 5599 | 2.0136 | 1.7081 | 1.0975 | 2.0714 |
| 4292 | 2.5439 | 2.4356 | 1.0567 | 2.0849 |
| 4318 | 3.9071 | 3.2385 | 2.2197 | 2.1325 |
| 4609 | 1.8993 | 1.6259 | 1.0177 | 1.9476 |
| 5048 | 2.785 | 2.7529 | 1.0541 | 1.7318 |
| 5111 | 4.2837 | 3.9742 | 1.9304 | 2.2694 |
| 64714 | 6.467 | 5.3825 | 3.6477 | 2.2541 |
| 5290 | 2.9954 | 3.04 | 1.0397 | 2.1615 |
| 5468 | 2.4615 | 2.319 | 1.0672 | 2.1334 |
| 11331 | 1.5531 | 1.1883 | 1 | 1.8861 |
| 6401 | 1.4903 | 1.1063 | 1 | 1.8262 |
| 4087 | 8.1384 | 7.0622 | 4.2476 | 2.151 |
| 4088 | 1.6512 | 1.3166 | 1 | 1.69 |
| 6647 | 10.376 | 8.4847 | 6.0317 | 1.6982 |
| 6774 | 4.1736 | 3.5526 | 2.2603 | 2.1934 |
| 6890 | 3.1265 | 3.0063 | 1.2834 | 2.288 |
| 7040 | 5.4139 | 5.8041 | 1.5112 | 2.3273 |
| 7076 | 1.6652 | 1.3349 | 1 | 1.7245 |
| 7124 | 3.3173 | 3.3289 | 1.1963 | 2.2127 |
| 7157 | 6.516 | 5.7608 | 3.2743 | 1.7914 |

Part XII: Evodiamine-related genes and corresponding $VC_D$ values

| Gene | $VC_D$ value | NodeRank | Betweenness | Closeness |
|---|---|---|---|---|
| 2353 | 2.4229 | 2.2352 | 1.1069 | 2.0575 |
| 885 | 1.9006 | 1.642 | 1.0006 | 1.7429 |
| 7422 | 3.8873 | 4.0189 | 1.2617 | 1.6696 |

(continued)

| Gene | VC$_D$ value | NodeRank | Betweenness | Closeness |
|------|--------------|----------|-------------|-----------|
| 796 | 1.4995 | 1.1183 | 1 | 1.6637 |
| 5743 | 1.9179 | 1.6651 | 1 | 1.4623 |
| 488 | 4.8152 | 4.8053 | 1.7683 | 1.8434 |
| 2520 | 9.1725 | 8.4393 | 4.2172 | 2.0956 |
| 7124 | 3.3173 | 3.3289 | 1.1963 | 2.2127 |
| 5594 | 7.0537 | 6.7568 | 2.9258 | 1.8812 |
| 3569 | 3.1151 | 3.2297 | 1.0001 | 1.4774 |
| 207 | 1.7431 | 1.4325 | 1.0049 | 1.9451 |
| 581 | 2.9385 | 2.8448 | 1.1833 | 1.9443 |
| 596 | 9.2204 | 7.9588 | 4.8626 | 2.2095 |
| 598 | 17.66 | 16.465 | 7.8613 | 2.3003 |
| 1543 | 1.4298 | 1.0272 | 1 | 1.9391 |
| 2048 | 4.543 | 4.4054 | 1.8208 | 2.3223 |
| 4843 | 8.071 | 7.9946 | 3.0349 | 2.4935 |
| 5243 | 3.2845 | 3.229 | 1.2641 | 2.105 |
| 1026 | 1.8209 | 1.5255 | 1.0152 | 1.9969 |
| 23411 | 1.721 | 1.4077 | 1 | 2.0809 |

Part XIII: Aconitine-related genes and corresponding VC$_D$ values

| Gene | VC$_D$ value | NodeRank | Betweenness | Closeness |
|------|--------------|----------|-------------|-----------|
| 6647 | 10.376 | 8.4847 | 6.0317 | 1.6982 |
| 4086 | 1.4476 | 1.0505 | 1 | 1.9562 |
| 4087 | 8.1384 | 7.0622 | 4.2476 | 2.151 |
| 4088 | 1.6512 | 1.3166 | 1 | 1.69 |
| 4089 | 35.049 | 29.09 | 19.867 | 2.6953 |
| 4090 | 2.2881 | 2.1047 | 1.0526 | 1.7937 |
| 5594 | 7.0537 | 6.7568 | 2.9258 | 1.8812 |
| 5243 | 3.2845 | 3.229 | 1.2641 | 2.105 |
| 1392 | 5.5977 | 5.838 | 1.7565 | 1.7954 |

Part XIV: Genistein-related genes and corresponding VC$_D$ values

| Gene | VC$_D$ value | NodeRank | Betweenness | Closeness |
|------|--------------|----------|-------------|-----------|
| 90 | 1.5204 | 1.1457 | 1 | 1.7457 |
| 207 | 1.7431 | 1.4325 | 1.0049 | 1.9451 |
| 836 | 1.5869 | 1.2325 | 1 | 1.7449 |
| 1028 | 2.6337 | 2.4422 | 1.1883 | 1.9279 |
| 1030 | 3.5809 | 2.9977 | 1.9992 | 1.6573 |
| 1385 | 2.3387 | 1.8236 | 1.4653 | 1.9964 |
| 2022 | 3.9549 | 3.8831 | 1.5281 | 2.1978 |
| 2064 | 2.7437 | 2.7444 | 1 | 1.6661 |
| 3761 | 1.4793 | 1.0918 | 1 | 2.0298 |
| 5594 | 7.0537 | 6.7568 | 2.9258 | 1.8812 |
| 1432 | 3.3638 | 2.9068 | 1.7701 | 1.959 |
| 5595 | 4.2418 | 3.3814 | 2.5698 | 1.9309 |

(continued)

| Gene | $VC_D$ value | NodeRank | Betweenness | Closeness |
|---|---|---|---|---|
| 9261 | 16.366 | 15.381 | 7.1406 | 2.2255 |
| 4846 | 11.262 | 8.6995 | 7.1534 | 2.4877 |
| 4086 | 1.4476 | 1.0505 | 1 | 1.9562 |
| 6714 | 1.8803 | 1.5874 | 1.034 | 2.0763 |

Part XV:genes relating to endothelial cell migration and corresponding $VC_D$ values

Note: gene products as recorded by GO0043542 (Homo sapiens) had the following gene product information of $VC_G$ value in angiogenesi gene network after redundant-removal

| Gene | $VC_D$ value | NodeRank | Betweenness | Closeness |
|---|---|---|---|---|
| 4763 | 2.8498 | 2.713 | 1.2021 | 1.7802 |
| 1906 | 6.7202 | 6.7117 | 2.4615 | 2.4201 |
| 7422 | 3.8873 | 4.0189 | 1.2617 | 1.6696 |
| 5155 | 2.2585 | 2.103 | 1.0086 | 1.7336 |
| 186 | 15.496 | 14.366 | 6.9949 | 2.4326 |
| 94 | 10.138 | 7.9944 | 6.2446 | 2.3473 |
| 1012 | 2.6677 | 2.5622 | 1.0985 | 1.8875 |
| 348 | 50.308 | 39.493 | 31.203 | 2.8194 |
| 5340 | 1.7132 | 1.3975 | 1 | 1.6504 |

Part XVI: matrix of shortest paths between genes of matrine - endothelial cell migration

(3,4,3,3,5,4,6,4,3);(3,3,2,2,3,2,4,3,2);(3,4,3,3,4,3,5,4,3);(3,4,2,2,3,3,5,3,3);(1,3,2,2,3,3,4,3,3);(3,3,3,3,4,3,4,3,4);(1,3,2,1,3,3,4,3,3);(3, 3,2,3,4,3,4,3,3);(3,3,3,3,4,2,5,4,3);(2,3,3,2,4,3,4,3,3);(3,2,3,2,4,4,5,2,2);(3,3,2,3,3,3,4,3,3);(3, 3,2,3,3,2,4,3,3);  (3,3,3,3,3,3,5,3,3);  (2,3,2 , 2,4,2,4,3,3);  (3,4,3,3,4,2,5,3,4);  (3,4,3,3,4,4,4,3,3);  (3,2,3,3,4,2,4,3,3); (3,3,3,3,4,3,5,3,3);  (3,3,1,2,3,3,4,3,3);  (3,3,2,2,3,3,4,3,3);  (2,3,3,3, 4,2,4,3,3);  (4,4,3,3,4,3,5,3,1);  (2,3,2,2,4,3,4,2,2); (3,3,3,3,3,2,4,3,3);  (4,4,4,4,4,5,6,4,4);  (3,4,2,2,3,3,4,3,2);  (3,3,3,3,3,3,4,3,2);  (3,3,2,2,4,2 , 5,4,3);  (4,5,4,4,4,5,6,4,3); (3,3,3,3,3,4,5,3,2);  (4,4,3,3,4,4,5,4,3);  (4,3,4,3,5,3,4,3,2);  (4,4,3,3,4,3,5,4,3);  (5,4,4,4,5,4,6,4,3);  (5,5,3,4,4,4,6, 5,4); (3,4,3,3,4,4,5,3,3);  (2,3,2,2,4,1,5,2,2);  (2,4,2,1,4,3,4,3,2);  (4,3,3,3,5,3,5,3,3);  (3,4,2,2,3,2,4,3,2);  (3,4,0,2,3,3,5,3,2); (3,4,2,2,3,4,5,4,3 );  (3,4,3,3,4,3,4,3,3);  (3,4,3,3,5,3,5,4,4);  (2,3,1,1,2,3,4,3,3);  (2,3,2,2,4,3,5,3,2);  (2,4,3,3,4,3,5,2,2); (3,4,3,2,4,2,5,2,2);  (2,3,2,2,4,3,5,3,2);  ( 2,3,2,1,3,3,4,1,2);  (3,4,2,2,3,3,5,4,3)

Part XVII: matrix of shortest paths between genes of quercetin-endothelial cell migration

(3,3,2,3,3,3,3,3,3);(3,4,3,3,4,3,4,3,2);(3,4,3,3,4,3,5,4,3);(3,4,3,3,3,3,4,3,3);(2,3,2,2,4,3,4,2,2);(2,4,3,3,4,3,5, 2,3);(4,5,3,4,4,4,4,4,2);(3, 3,3,2,4,3,3,3,2);(3,3,2,3,4,3,4,3,3);(3,3,3,3,3,2,4,3,3);(2,4,2,1,4,3,4,3,2);(4,5,4,4,5,4,5,5,4); (4,5,4,4,5,4,5,5,4);(3,3,3,3,4,3,5,4,3);(3,3,1 ,1,2,3,4,3,3);(3,3,2,2,1,4,4,3,3);(2,4,2,2,4,2,4,3,3);(3,3,1,1,4,2,4,3,3); (3,4,2,2,3,3,5,4,3);(3,4,3,2,4,3,5,2,2);(3,4,3,3,5,3,5,4,4);(3,4,3,3, 4,3,5,3,2);(2,3,2,2,4,2,4,3,3);(2,3,2,2,3,2,3,2,3);(3,4,2, 3,3,2,4,3,3);(2,3,2,2,4,2,3,3,3);(2,3,3,2,4,3,5,3,3);(2,2,3,2,3,3,4,3,3);(3,3,3,3,4,3 ,5,3,3);(2,4,2,2,4,3,4,3,3);(4,4,3,3, 3,3,5,4,3);(3,3,3,3,4,2,4,3,3);(3,3,1,2,3,3,4,3,3);(3,4,3,3,4,3,4,3,3);(3,3,2,2,3,2,4,3,2);(3,4,3,3,3,4,4, 3,3);(3,4,0,2,3,3, 5,3,2)

Part XVIII: matrix of shortest paths between genes of emodin-endothelial cell migration

(3,2,3,2,4,4,5,2,2);(2,3,3,2,4,3,4,3,3);(3,4,3,3,4,4,5,3,3);(3,3,3,3,4,3,5,3,3);(2,3,2,2,4,3,4,2,2);(4,5,3,4,4,4, 4,4,2);(4,4,3,3,4,3,5,4,3);(3, 4,3,3,4,2,5,3,4);(3,3,3,3,4,2,5,4,3);(3,3,3,3,4,3,4,3,4);(3,3,3,3,3,4,5,3,3);(3,4,2,2,3,4,5,3,3); (2,3,2,2,4,3,5,3,2);  (2,4,2,2,4,2,4,3,3);  (3,3,2 , 3,4,2,4,3,3);  (3,4,3,3,4,3,4,4,4);  (4,4,3,2,5,4,6,2,2);  (3,3,2,2,3,3,4,3,3); (3,3,2,2,3,3,4,3,3);(2,3,2,2,4,2,4,3,3);(1,3,2,2,3,3,4,3,3);(3,3,2,3, 3,3,4,3,3);(3,4,3,3,4,3,5,3,3);(4,4,3,3,4,3,5,3,3);(2,3,2, 2,3,2,3,2,3);  (3,4,2,3,3,2,4,3,3);  (2,3,2,2,4,2,3,3,3);  (3,4,3,3,4,3,5,3,3);  (3,4,3,2,4,3 , 5,3,1);  (3,3,2,3,3,2,4,3,3); (4,4,4,4,4,4,5,4,4);  (3,3,3,3,3,3,5,3,3);  (3,4,3,3,4,4,4,3,3);  (2,4,2,2,2,3,3,3,3);  (3,4,3,3,4,3,2,3,3);  (4,4,3,3,4,3,4, 4,4); (4,4,3,3,3,3,5,4,3);  (3,3,3,3,4,1,4,3,2);  (2,3,2,2,4,1,4,3,2);  (3,4,3,3,4,3,5,4,3);  (3,3,1,2,3,3,4,3,3);  (4,4,3,3,4,3,5,4,4); (2,3,2,2,4,1,5,2,2 );  (4,3,4,3,5,3,4,3,2);  (3,4,3,3,4,3,4,3,3);  (3,3,2,2,3,2,4,3,2)

Part XIX: matrix of shortest paths between genes of evodiamine - endothelial cell migration

(2,4,2,2,4,2,4,3,3);(4,4,4,4,6,5,7,4,2);(3,4,0,2,3,3,5,3,2);(6,3,5,5,6,6,7,6,5);(3,3,3,3,4,3,5,3,3);(3,4,3,3,4,4,5,
4,4);(4,2,4,4,4,5,6,4,2);(3,   4,3,3,4,3,4,3,3);(2,3,2,2,3,2,3,2,3);(3,4,3,3,5,3,5,4,4);(3,3,2,3,3,3,3,3,3);(3,4,3,3,4,3,5,4,3);
(2,3,3,2,4,3,4,3,3);  (3,4,3,3,3,3,4,3,3);  (4,5,4 ,  4,5,4,5,5,4);  (2,4,3,3,3,5,3,3);  (2,3,3,2,4,3,5,3,3);  (3,3,3,3,4,3,5,3,3);
(3,3,2,3,4,3,4,3,3);(4,3,3,3,4,3,4,4,3)

Part XX: matrix of shortest paths between genes of aconitine - endothelial cell migration

(3,4,3,3,4,3,5,4,3);(3,3,3,3,4,2,4,3,3);(3,3,3,3,4,1,4,3,2);(2,3,2,2,4,1,4,3,2);(2,3,3,3,4,2,4,3,3);(3,4,3,3,4,3,4,
3,4);(2,3,2,2,3,2,3,2,3);(3,  3,3,3,4,3,5,3,3);(5,4,4,4,5,4,7,5,4)

Part XXI: matrix of shortest paths between genes of genistein - endothelial cell migration

(2,4,3,3,4,2,5,4,3);(3,3,2,3,3,3,3,3);(2,3,2,2,4,3,4,2,2);(3,3,3,3,4,2,5,4,3);(3,3,3,3,4,2,5,4,3);(3,3,3,3,4,3,4,
3,4);(3,4,3,3,5,2,6,3,3);(3,   3,1,1,2,3,4,3,3);(4,5,4,4,5,5,6,4,4);(2,3,2,2,3,2,3,2,3);(3,4,2,3,3,2,4,3,3);(2,3,2,2,3,2,4,2,3);
(3,3,3,3,3,3,4,3,3);(2,2,3,2,3,3,4,3,3);(3,3,3 ,3,4,2,4,3,3);(2,3,2,2,3,3,4,2,2)
While the invention has been described with the above embodiments, the above description is not given for
limitation of the invention. For example, although at least one of the two gene subsets (two processes) in the above
embodiments corresponds to action of medicine, the present invention is not limited to this. In the situation that all the
two gene subsets/processes are pathologic/physiological/pharmacological/toxic/side-effect processes, the present invention can be implemented as a method for determining the mechanism of and/or relationship among the pathologic/
physiological/pharmacological/toxic/side-effect processes. Such variations, as well as other changes, modifications, and
improvements, are within the scope of the present invention.

**Claims**

1.   a method for determining a medicine action on the basis of gene network, **characterized by** comprising:

    determining at least one network topological attribute of a first subset of genes/gene products with respect to
    a second subset of genes/gene products in a gene network,
    where
    said first subset of genes/gene products includes genes/gene products relating to a first medicine,
    said second subset of genes/gene products include genes/gene products relating to at least one of the following:

        a second medicine, and
        a bioprocess.

2.   a method as claimed in Claim 1, **characterized in that** said at least one network topological attribute comprises:

    the length of the shortest network path in said gene network between said first subset of genes/gene products
    and said second subset of genes/gene products, and
    node importance of genes/gene products of said first subset of genes/gene products and/or genes/gene products
    of said second subset of genes/gene products.

3.   a method as claimed in Claim 2, **characterized by** comprising performing comprehensive processing to at least
    one parameter in the following parameters of each of at least some of the nodes in said gene network so as to obtain
    a node importance factor (IP, VC) indicating the importance of said node:- a parameter (NodeRank) indicating an
    eigenvector corresponding to the maxiumu eigenvalue of a network-associated matrix of said gene network,

        - a parameter (Betweenness) indicating the number of shortest paths among all genes in said gene network
        which go through said node, and
        - a parameter (Closeness) indicating the inverse of the sum of the shortest paths from said node to each of all
        other nodes in the network.

4.   a method as claimed in Claim 2, wherein said gene network is one selected from:

a network relating to a disease constructed on the basis of literature information and/or enome, transcriptome, proteome, and/or metabolomics data relating to said disease,
a network formed by all the network relationships existing in a public protein-protein interaction network database, and
a network formed by all the network relationships existing in a public pathway database.

5. a method as claimed in Claim 2, **characterized by** further comprising:

constructing a disease-related gene network of said disease, comprising:

for each pair/group of genes/gene products in a known database of genes/gene products, determining whether all genes/gene products in said pair/group of genes/gene products are included in a set of genes/gene products relating to said disease obtained by analysis based on literature and/or experimental information, and
when all genes in said pair/group appear in said set of genes/gene products relating to said disease, determining that the genes in said pair/group of genes are adjacent genes in said disease-related gene network.

6. a method as claimed in Claim 3, **characterized by** that said comprehensive processing comprises principal components analysis.

7. a method as claimed in Claim 2, **characterized in that**
the shortest network path is determined by using Floyd Algorithm,
and wherein
said parameter (NodeRank) indicating an eigenvector corresponding to the maxiumu eigenvalue of a network-associated matrix of said gene network indicates:

$$P(A) = \frac{1-d}{N} + d \sum_{v \in L_v} \frac{P(v)}{N_v}$$

where N is the number of all nodes in the network, d is an attenuating factor smaller than 1, which indicates uncertainty of edges in the network, $L_v$ is the set of nodes which directly connects to node A, and $N_v$ is the number of edges of node $v$;
said parameter (Betweenness) indicating the number of shortest paths among all nodes in said gene network which go through said gene indicates

$$C_B(w) = \sum_{s \in V} \sum_{d \neq s \in V} \frac{\sigma_{sd}(w)}{\sigma_{sd}}$$

where $\sigma_{sd}$ is the number of shortest paths between any two nodes in said gene network, $\sigma_{sd}(W)$ is the number of paths among said shortest paths which go through node w, and $V$ is the set of nodes in said network which connects to node $v$, and
said parameter (Closeness) indicating the inverse of the sum of the shortest paths from said gene to each of all other genes in the network indicates:

$$C(v) = \frac{1}{\sum_{t \in V} d_{v,t}}$$

where $V$ is the set of nodes in said network which connects to node $v$, $d_{v,t}$ is the shortest path from node $v$ to node $t$.

8. a method as claimed in Claim 2, **characterized by** using a value indicating a normalized weighted average of said node of said node importance factor (IP, VC) to characterize said network topological attribute, wherein the weight of said weighted average is inversely correlated with the minimum of the length of the shortest network path in said gene network between said first subset of genes/gene products and said second subset of genes/gene products.

## Fig.1a

Inhibition rate(%)

[S]:[M] = 1:10
Bliss additive effect

## Fig.1b

Inhibition rate(%)

[S]:[H] = 5:1
Bliss additive effect

## Fig.1c

Inhibition rate(%)

[S]:[L] = 5:1
Bliss additive effect

## Fig.1d

Inhibition rate(%)

[S]:[Q] = 5:1
Bliss additive effect

## Fig.1e

Inhibition rate(%)

[S]:[P] = 5:1
Bliss additive effect

## Fig.1f

MIIR (%)

SM SH SL SQ SP

Fig .2

# Fig.3

Fig.4a

a. 0h

Fig.4b

b. Control group 9h

Fig.4C

c. Matrine 9h

Fig.4d

d. Quercetin 9h

Fig.4e

**e. Emodin 9h**

Fig.4f

**f. Evodiamine 9h**

Fig.4g

**Genistein 9h**

Fig.4h

**h. Aconitine 9h**

Fig.4i

Form PCT/ISA /210 (continuation of first sheet (3)) (April 2007)

# INTERNATIONAL SEARCH REPORT

International application No.

PCT/CN2009/074377

**A.   CLASSIFICATION OF SUBJECT MATTER**

See extra sheet

According to International Patent Classification (IPC) or to both national classification and IPC

**B.    FIELDS SEARCHED**

Minimum documentation searched (classification system followed by classification symbols)

G06F17/30,G06F17/00,G06F 17/15,G06F19/00,C12N 15/00,C40B 40/06

Documentation searched other than minimum documentation to the extent that such documents are included in the fields searched

Electronic data base consulted during the international search (name of data base and, where practicable, search terms used)

WPI, EPODOC, CNKI, CNPAT, Web of Knowledge, Elsevier Science Direct,Wiley InterScience,Nature,Sciene,Embase, network,disease,drug,medicine,pharmaceutical,gene,synergistic,map,topology,nod,distance,pathway

**C.   DOCUMENTS CONSIDERED TO BE RELEVANT**

| Category* | Citation of document, with indication, where appropriate, of the relevant passages | Relevant to claim No. |
|---|---|---|
| X | Y1LD1R1M,Muhammed A et al. Drug-target network，NATURE BIOTECHNOLOGY，Oct.2007（10.2007）,vol.25,no.10,pages 1119-1126 | 1 |
| A | LI, Shao　Framework and practice of network-based studies for Chinese herbal formula, JOURNAL OF CHINESE INTEGRATIVE MEDICINE, Sep.2007(09.2007),vol.5,no.5,pages 489-493 | 1-8 |
| A | ZHANG, Zhizhou　Formula western drug maybe one of final strategic aims of modernization of traditional Chinese medicine, CHINA BIOTECHNOLOGY,2007(2007),vol.27,no.4,pages 153-156 | 1-8 |

☒　Further documents are listed in the continuation of Box C.　　☒　See patent family annex.

| | |
|---|---|
| *　　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim (S) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"document member of the same patent family |

| Date of the actual completion of the international search | Date of mailing of the international search report |
|---|---|
| 24 Dec.2009（24.12.2009） | **14 Jan. 2010 (14.01.2010)** |

| Name and mailing address of the ISA/CN The State Intellectual Property Office, the P.R.China 6 Xitucheng Rd., Jimen Bridge, Haidian District, Beijing, China 100088 Facsimile No. 86-10-62019451 | Authorized officer　Zhang,Xiuli Telephone No. (86-10)62413896 |

Form PCT/ISA /210 (second sheet) (July 2009)

# INTERNATIONAL SEARCH REPORT

| | International application No. |
|---|---|
| | PCT/CN2009/074377 |

C (Continuation).    DOCUMENTS CONSIDERED TO BE RELEVANT

| Category* | Citation of document, with indication, where appropriate, of the relevant passages | Relevant to claim No. |
|---|---|---|
| A | BILLINGSLEY, Melvin L. Druggable targets and targeted drugs: enhancing the development of new therapeutics，PHARMACOLOGY，19 Sep.2008（19.09.2008），vol.82, pages239-244 | 1-8 |
| A | NIKOLSKY, Y. et al.Biological networks and analysis of experimental data in drug discovery，May 2005（05.2005），DRUG DISCOVERY TODAY,vol.10,no.9,pages 653-662 | 1-8 |
| A | HOPKINS, Andrew L Network pharmacology:the next paradigm in drug discovery，NATURE CHEMICAL BIOLOGY, Nov.2008（11.2008），vol.4,no.1,pages682-690 | 1-8 |
| A | HWANG ,W-C et al.，Identification of information flow-modulating drug targets: a novel bridging paradigm for drug discovery，CLINICAL PHARMACOLOGY & THERAPEUTICS，Nov.2008（11.2008），vol.85,no.5,pages563-572 | 1-8 |
| A | PITLUK,Zach et al.Achieving confidence in mechanism for drug discovery and development，DRUG DISCOVERY TODAY, Nov.2007（11.2007），vol.12,no.21/22,pages 924-930 | 1-8 |
| A | US20020077756A1（AROUH, Scott et al），20 Jan.2002（20.01.2002），the whole document | 1-8 |
| A | US20050241010A1(GREENSPAN,Ralph J. et al),27 Oct.2005（27.10.2005），the whole document | 1-8 |
| A | US20070038385A1(NIKOLSKAYA, Tatiana et al),15 Feb.2007（15.02.2007），the whole document | 1-8 |
| A | WO2006058014A2(ODYSSEY THFRA,INC.)，01 Jun.2006(01.06.2006), the whole document | 1-8 |

Form PCT/ISA /210 (continuation of second sheet ) (July 2009)

## INTERNATIONAL SEARCH REPORT
Information on patent family members

| International application No. |
| --- |
| PCT/CN2009/074377 |

| Patent Documents referred in the Report | Publication Date | Patent Family | Publication Date |
| --- | --- | --- | --- |
| US20020077756A1 | 20.01.2002 | None | |
| US20050241010A1 | 27.10.2005 | None | |
| US20070038385A1 | 15.02.2007 | None | |
| WO2006058014A2 | 01.06.2006 | EP1836631A2 | 26.09.2007 |
| | | US2006160109A1 | 20.07.2006 |
| | | AU2005309649A1 | 01.06.2006 |
| | | CA2590331A1 | 01.06.2006 |

Form PCT/ISA /210 (patent family annex) (July 2009)

## INTERNATIONAL SEARCH REPORT

International application No.

PCT/CN2009/074377

Continuation of: CLASSIFICATION OF SUBJECT MATTER

G06F17/30(2006.01)i
G06F17/00(2006.01)i
G06F17/15(2006.01)i
G06F19/00(2006.01)i
C12N15/00(2006.01)i
C40B40/06(2006.01)i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• CN 200610114226X

### Non-patent literature cited in the description

• **Li S ; Wu LJ ; Zhang ZQ.** Constructing biological networks through combined literature mining and microarray analysis: a LMMA approach. *Bioinformatics,* 2006, vol. 22, 2143-2150

• **Van Driel, M.A. et al.** A Text-Mining Analysis of the Human Phenome. *Eur. J. Hum. Genet.,* 2006, vol. 14, 535-542